# EUROPEAN PATENT SPECIFICATION

(11) **EP 4 133 966 B1**
(45) Date of publication and mention of the grant of the patent: **23.07.2025**
(21) Application number: 20930567.1
(22) Date of filing: 11.09.2020
(51) Int. Cl.: A45D 20/12, A45D 44/00, A61H 39/04, A61B 5/053, A61B 5/00, A61N 5/06

(54) **DIFFUSER AND HAIR DRYER HAVING SAME**
DIFFUSOR UND HAARTROCKNER, DER EINEN SOLCHEN DIFFUSOR UMFASST
DIFFUSEUR ET SÈCHE-CHEVEUX PRÉSENTANT CE DERNIER

(30) Priority: 10.04.2020 KR 20200044036
(43) Date of publication of application: 15.02.2023
(73) Proprietor: LG Electronics, Inc., Yeongdeungpo-gu Seoul 07336 (KR)
(72) Inventor: KIM, Kyoungtae, Seoul 08592 (KR); KIM, Hyunchul, Seoul 08592 (KR); KU, Yunhee, Seoul 08592 (KR); PARK, Rayoung, Seoul 08592 (KR); KIM, Dongwon, Seoul 08592 (KR)
(74) Representative: Vossius & Partner Patentanwälte Rechtsanwälte mbB
(86) International application number: PCT/KR2020/012273
(87) International publication number: WO 2021/206229

(56) References cited:
- CN-A- 109 527 764
- JP-A- 2018 175 726
- JP-A- 2018 175 726
- KR-A- 20100 066 043
- KR-A- 20180 017 193
- KR-A- 970 706 745
- KR-B1- 101 155 135
- KR-B1- 101 872 227
- KR-B1- 101 872 227

## Description

### [Technical Field]

The present disclosure relates to a diffuser and a hair dryer including the same, more particularly, to a diffuser coupled to a main body of a hair dryer to provide gas to a user and the hair dryer including the same.

### [Background Art]

When removing moisture from wet hair of a human body as much as desired or when styling the hair from a current shape to a desired shape, a hair dryer that discharges gas through a gas outlet may be used.

In one example, the hair dryer may provide gas characteristics desired by a user, such as a gas temperature, a gas speed, a gas flow area, and the like through a diffuser. The diffuser may be coupled to a main body of the hair dryer to change the gas characteristics and provide the changed gas characteristics to the user. Further, the diffuser may include care means such as a massage protrusion to manage a scalp health and the like of the user.

In connection, Korean Utility Model Application Publication No. 20-2011-0002484 and Chinese Application CN 109 527 764 disclose a diffuser disposed in a hair dryer. The diffuser disclosed in the Korean document discloses a massage protrusion that may perform user's scalp and hair care.

The diffuser may include various components to manage or care for scalp and hair in addition to the massage protrusion.

In one example, depending on conditions of the user's scalp and hair, uniform application of the means provided for the management of the user's scalp or hair may degrade efficiency or ease of use.

Therefore, it is an important task in the technical field to provide various means for the management and care of the user's scalp or hair, and further to provide appropriate care means based on the conditions of the user's scalp or hair.

### [Disclosure]

### [Technical Problem]

Embodiments of the present disclosure are intended to provide a diffuser and a hair dryer including the same capable of effectively managing scalp and hair of a user.

In addition, embodiments of the present disclosure are intended to provide a diffuser and a hair dryer capable of effectively identifying conditions of user's scalp and hair.

In addition, embodiments of the present disclosure are intended to provide a diffuser and a hair dryer capable of improving ease of use and efficiency by providing appropriate care means based on conditions of user's scalp and hair.

### [Technical Solution]

A diffuser and a hair dryer including the same according to an embodiment of the present disclosure may include scalp and hair care means. The care means may include a massage protrusion, a light irradiator, and a controller that controls speed and temperature of gas.

An embodiment of the present disclosure may include moisture measurement means capable of measuring moisture amounts of scalp and hair of a user. The moisture measurement means may be disposed in a form of a massage protrusion disposed on a discharge cover of the diffuser.

That is, in an embodiment of the present disclosure, some of a plurality of massage protrusions may correspond to moisture measurement protrusions capable of measuring the moisture amounts of the scalp and the hair of the user. The moisture amount of the scalp may be measured through the moisture measurement protrusion to provide the appropriate care means.

There are limitations in using a common hygrometer or moisture sensor to measure the moisture of the user's scalp or hair. Accordingly, an embodiment of the present disclosure may provide a moisture measurement technology optimized for the moisture measurement of the scalp or the hair.

In an embodiment of the present disclosure, a voltage may be generated as the moisture measurement protrusion is connected to the light irradiator, so that the moisture measurement protrusion may measure the moisture. That is, the voltage may be generated of a measured value may be transmitted as the moisture measurement protrusion is connected to a circuit board of the light irradiator.

The moisture measurement protrusion may measure the moisture amount of the scalp or the like based on a principle of a bioelectric impedance that changes based on the moisture amount.

When sensing through the moisture measurement protrusion starts, a voltage is applied to the moisture measurement protrusion and an electric field is formed due to polarity occurrence resulted from the voltage generation. The electric field is scattered along the scalp.

As the moisture amount increases, the electric field is amplified and an electrical capacity increases at the scalp and the like on which the electric field is formed. The controller may calculate and determine the moisture amount based on measured values for the change as described above.

The controller may determine whether to operate the light irradiator, control a temperature of the gas, or control a speed of the gas based on the moisture amount.

The hair dryer according to an embodiment of the present disclosure as described above may include a main body, a handle, and a diffuser. The main body includes a gas outlet for discharging gas therethrough, the handle extends from the main body, and the diffuser is removably coupled to the main body to introduce the gas discharged from the gas outlet therein and discharge the gas introduced therein to outside.

The diffuser includes a diffusing case and a discharge cover. The diffusing case has a rear side coupled to the main body, and the gas discharged from the gas outlet is introduced into the diffusing case through a gas inlet hole defined at the rear side. **In** addition, the discharge cover is disposed at a front side of the diffusing case, and the discharge cover includes a gas discharge hole for discharging the gas introduced into the diffusing case to outside.

The discharge cover includes a plurality of massage protrusions protruding forward to press a target located in front of the discharge cover, and the plurality of massage protrusions include a moisture measurement protrusion disposed to measure a moisture amount of the target.

The moisture measurement protrusion may include a protrusion base protruding forward to press the target, and a moisture measurement electrode disposed in the protrusion base, wherein at least a portion of the moisture measurement electrode is exposed out of the protrusion base, and wherein the moisture measurement electrode has an electrical polarity as a voltage is applied thereto.

The moisture measurement electrode may extend to penetrate the protrusion base along a longitudinal direction of the protrusion base and may be exposed to outside through an end of the protrusion base.

The moisture measurement protrusion may include a plurality of moisture measurement protrusions, the plurality of moisture measurement protrusions may include a first moisture measurement protrusion including a moisture measurement electrode having a first pole, and a second moisture measurement protrusion including a moisture measurement electrode having a second pole opposite to the first pole, and the first moisture measurement protrusion and the second moisture measurement protrusion may be arranged to be adjacent to each other.

The plurality of massage protrusions may include a plurality of pairs of moisture measurement protrusions, wherein each pair of moisture measurement protrusions includes the first moisture measurement protrusion and the second moisture measurement protrusion.

The hair dryer may further include a controller electrically connected to the plurality of moisture measurement protrusions, wherein the controller may determine the moisture amount based on an impedance measured between the first moisture measurement protrusion and the second moisture measurement protrusion.

The hair dryer may further include a temperature adjuster disposed on the main body to adjust a temperature of the gas discharged through the gas outlet, wherein the controller may control the temperature adjuster such that the temperature of the gas discharged through the gas outlet increases as the moisture amount increases.

The hair dryer may further include a fan disposed to adjust a speed of the gas discharged through the gas outlet, wherein the controller may control the fan such that the speed of the gas discharged through the gas outlet increases as the moisture amount increases.

The hair dryer may further include a light irradiator disposed inside the diffusing case to irradiate light toward the discharge cover, wherein the controller may control the light irradiator such that an amount of the light irradiated by the light irradiator increases as the moisture amount increases.

The light irradiator may include a circuit board and a light emitter disposed on the circuit board to emit light, and the moisture measurement electrode may be connected to the circuit board to receive the voltage.

The light irradiator may include a plurality of light emitters arranged to respectively face the plurality of massage protrusions.

**In** one example, a diffuser according to an embodiment of the present disclosure may include a diffusing case having a rear side removably coupled to a main body of a hair dryer, wherein gas discharged from the main body is introduced into the diffusing case through a gas inlet hole defined at the rear side, and a discharge cover disposed at a front side of the diffusing case, wherein the discharge cover includes a gas discharge hole for discharging the gas introduced into the diffusing case to outside.

The discharge cover may include a plurality of massage protrusions protruding forward to press a target located in front of the discharge cover, and the plurality of massage protrusions may include a moisture measurement protrusion disposed to measure a moisture amount of the target.

The moisture measurement protrusion may include a protrusion base protruding forward to press the target, and a moisture measurement electrode disposed in the protrusion base, wherein at least a portion of the moisture measurement electrode is exposed out of the protrusion base, and wherein the moisture measurement electrode has an electrical polarity as a voltage is applied thereto.

The moisture measurement protrusion may include a plurality of moisture measurement protrusions, the plurality of moisture measurement protrusions may include a first moisture measurement protrusion including a moisture measurement electrode having a first pole, and a second moisture measurement protrusion including a moisture measurement electrode having a second pole opposite to the first pole, and the first moisture measurement protrusion and the second moisture measurement protrusion may be arranged to be adjacent to each other.

The plurality of massage protrusions may include a plurality of pairs of moisture measurement protrusions, wherein each pair of moisture measurement protrusions includes the first moisture measurement protrusion and the second moisture measurement protrusion.

### [Advantageous Effects]

Embodiments of the present disclosure may provide the diffuser and the hair dryer including the same capable of effectively managing the scalp and the hair of the user.

In addition, embodiments of the present disclosure may provide the diffuser and the hair dryer capable of effectively identifying the conditions of the user's scalp and hair.

In addition, embodiments of the present disclosure may provide the diffuser and the hair dryer capable of improving the ease of use and the efficiency by providing the appropriate care means based on the conditions of the user's scalp and hair.

### [Description of Drawings]

FIG. 1 is a view showing a hair dryer according to an embodiment of the present disclosure;
FIG. 2 is a view showing a state in which a diffuser is separated from a hair dryer according to an embodiment of the present disclosure;
FIG. 3 is a view showing an internal cross-section of a hair dryer shown in FIG. 2;
FIG. 4 is a view showing a gas outlet in a hair dryer according to an embodiment of the present disclosure;
FIG. 5 is a view showing a diffuser according to an embodiment of the present disclosure;
FIG. 6 is a view showing an exploded view of a diffuser according to an embodiment of the present disclosure;
FIG. 7 is a view showing an internal cross-section of a diffuser according to an embodiment of the present disclosure;
FIG. 8 is a view showing a diffuser according to an embodiment of the present disclosure viewed from the front;
FIG. 9 is a view showing a moisture measurement protrusion connected to a light irradiator in a diffuser according to an embodiment of the present disclosure;
FIG. 10 is a view schematically showing a state in which a moisture amount is measured by a moisture measurement protrusion in an embodiment of the present disclosure; and
FIG. 11 is a view showing an arrangement relationship between a light emitter and a massage protrusion in a diffuser according to an embodiment of the present disclosure.

### [Best Mode]

Hereinafter, an embodiment of the present disclosure will be described in detail with reference to the accompanying drawings to be easily implemented by those skilled in the art to which the present disclosure belongs.

However, the present disclosure may be implemented in many different forms and is not limited to embodiments described herein. In addition, in order to clearly describe the present disclosure, components irrelevant to the description are omitted, and like reference numerals are assigned to similar components throughout the specification.

In this specification, duplicate descriptions of the same components are omitted.

Further, in this specification, it will be understood that when a component is referred to as being "connected with" another component, the component may be directly connected with the other component or intervening components may also be present. In contrast, it will be understood that when a component is referred to as being "directly connected with" another component in this specification, there are no intervening components present.

Further, in this specification, the terminology used herein is for the purpose of describing a specific embodiment only and is not intended to be limiting of the present disclosure.

Further, in this specification, the singular forms "a" and "an" are intended to include the plural forms as well, unless the context clearly indicates otherwise.

Further, in this specification, it will be further understood that the terms "comprises", "comprising", "includes", and "including" specify the presence of the certain features, numbers, steps, operations, elements, and parts or combinations thereof, but do not preclude the presence or addition of one or more other features, numbers, steps, operations, elements, and parts or combinations thereof.

Further, in this specification, the term 'and/or' includes a combination of a plurality of listed items or one of the plurality of listed items. In this specification, 'A or B' may include 'A', 'B', or 'both A and B'.

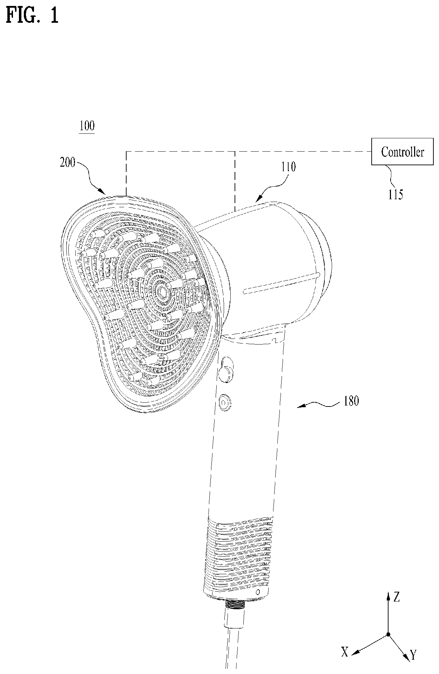

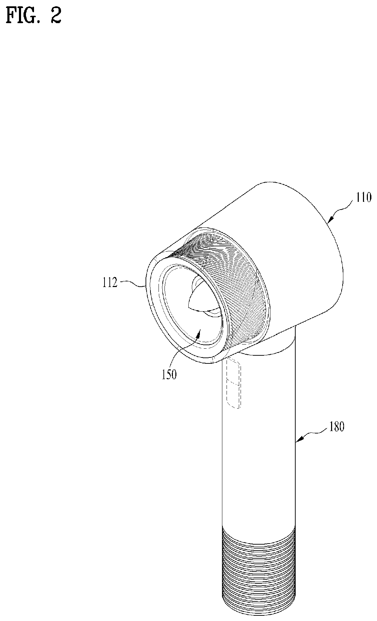

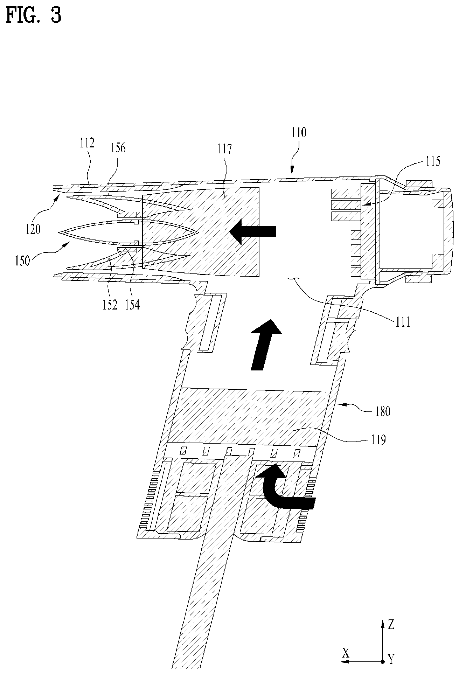

FIG. 1 is a view showing a hair dryer 100 according to an embodiment of the present disclosure. FIG. 2 is a view showing a state in which a diffuser 200 is separated from the hair dryer 100 shown in FIG. 1. Further, FIG. 3 is a view showing an internal cross-section of the hair dryer 100 shown in FIG. 2.

The hair dryer 100 according to an embodiment of the present disclosure includes a main body 110, a handle 180, and a diffuser 200 as shown in FIG. 1. In addition, as shown in FIG. 2, the main body 110 includes a gas outlet 150 through which gas introduced from outside is discharged.

As shown in FIG. 3, the main body 110 may include a gas flow path 111 through which the gas flows may be defined therein and the gas outlet 150 through which internal gas is discharged to the outside. The main body 110 may have an extended shape along a front and rear direction and may have various cross-section shapes such as circular or polygonal shapes when viewed from the front.

In the present disclosure, front, rear, left, right, top, and bottom definitions may be made centering on the main body 110. For example, referring to FIG. 2, the gas outlet 150 may be disposed at a front side of the main body 110, and the handle 180 may have a shape extending substantially downward from the main body 110.

The gas flowing inside the main body 110 may be introduced through a gas inlet, and the gas inlet may be disposed on the main body 110 or the handle 180. As shown in FIGS. 1 to 3, when the gas inlet is disposed on the handle 180, the gas flow path 111 may extend from the handle 180 to the main body 110, specifically, from the gas inlet to the gas outlet 150.

The gas may be introduced from the outside through the gas inlet disposed on the main body 110 or the handle 180, and the introduced gas may flow along the gas flow path 111 and be discharged to the outside through the gas outlet 150.

The handle 180 may extend from the main body 110. Referring to FIGS. 1 to 3, the handle 180 extending substantially downward from the main body 110 is illustrated. The handle 180 may be integrally molded with the main body 110, or separately manufactured from the main body 110 and coupled to the main body 110.

When the handle 180 is manufactured separately from the main body 110 and coupled to the main body 110, the handle 180 may be disposed such that a longitudinal direction thereof with respect to the main body 110 is fixed or variable.

For example, the handle 180 may have a hinge coupling portion, and may be coupled to the main body 110 such that the longitudinal direction of the handle 180 is changeable, that is, the handle 180 is foldable relative to the main body 110.

The handle 180 may be a portion grabbed by a hand of a user, and thus may have a shape for improving grip convenience. The extending direction of the handle 180 may vary. However, for convenience of description below, the direction in which the handle 180 extends from the main body 110 will be described as a downward direction.

Referring to FIG. 3, the hair dryer 100 according to an embodiment of the present disclosure includes a fan 119 capable of flowing the gas and adjusting a speed of the discharge gas discharged through the gas outlet 150. The fan 119 may be disposed on the gas flow path 111 to flow the gas and may be disposed inside the main body 110 or inside the handle 180.

For example, when the gas inlet is disposed on the handle 180, the gas flow path 111 may extend from the gas inlet of the handle 180 to the gas outlet 150 of the main body 110, and the fan 119 may be disposed on the gas flow path 111 located in the handle 180.

In addition, a temperature adjuster 117 that may adjust a temperature of the discharge gas may be disposed inside the main body 110. The temperature adjuster 117 may be disposed in various forms and may be disposed at various positions. In FIG. 2, the temperature adjuster 117 disposed inside the main body 110 is schematically illustrated.

In addition, the temperature adjuster 117 may be disposed in various types. The temperature adjuster 117 may be in a scheme of heating the gas by providing current to a coil-shaped resistor to generate heat.

However, the resistor of the temperature adjuster 117 may not necessarily be in the shape of the coil, and may be disposed in various types, such as a thermoelement, capable of heating the gas or adjusting the temperature of the gas.

A method for operating the hair dryer 100 according to an embodiment of the present disclosure will be schematically described with gas flow.

First, the user manipulates a power button disposed on the main body 110 or the handle 180. When the power button is turned on, the gas is introduced into the hair dryer 100 through the gas inlet as the fan 119 is operated.

The gas introduced through the gas inlet flows along the gas flow path 111 by the fan 119 toward the gas outlet 150, and the discharge gas is discharged from the gas outlet 150 to be provided to the user. In this process, a flowing speed of the gas on the gas flow path 111 may be adjusted by the fan 119 and a temperature of the gas on the gas flow path 111 may be adjusted by the temperature adjuster 117.

In one example, referring to FIGS. 1 and 2, the hair dryer 100 according to an embodiment of the present disclosure may include a controller 115. The controller 115 may be connected not only to the fan 119, the temperature adjuster 117, the power button, and a manipulator, but also to a light irradiator 260, a proximity sensor 269, a moisture measurement protrusion 312, and the like of the diffuser 200 to be described later, and control the above described components.

The controller 115 may be disposed on one of the diffuser 200, the main body 110, and the handle 180. Alternatively, the controllers 115 may be respectively arranged on all of the diffuser 200, the main body 110, and the handle 180. For example, as shown in FIG. 2, the controller 115 may be disposed on the main body 110 to be signally connected to the diffuser 200, or the plurality of controllers 115 may be respectively arranged on the diffuser 200 and the main body 110.

Adjusting operating states of the fan 119 and the temperature adjuster 117 may be performed by manipulator manipulation by the user or may be automatically performed based on an operation mode preset in the controller 115.

In addition, when a distance to a target located in front of the diffuser 200 is identified to be equal to or less than a reference distance through the proximity sensor 269 of the diffuser 200, the controller 115 may control the light irradiator 260 of the diffuser 200 to irradiate light.

In addition, the controller 115 may identify an impedance of the target located in front of the diffuser 200 through the moisture measurement protrusion 312 of the diffuser 200, and determine a moisture amount of the target through the impedance. As the moisture amount increases, the controller 115 may control the fan 119 such that the gas speed at the gas outlet 150 increases, control the temperature adjuster 117 such that the gas temperature increases, or control the light irradiator 260 such that a light amount of the light irradiator 260 increases.

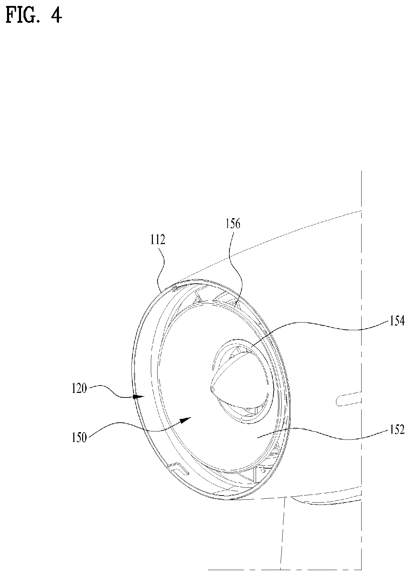

In one example, FIG. 4 shows the gas outlet 150 disposed on the main body 110. As shown in FIG. 1 or 3, the main body 110 may have a cross-section in an approximately circular shape and may have a length. However, the cross-section shape of the main body 110 may be varied as needed.

The gas outlet 150 of the hair dryer 100 according to an embodiment of the present disclosure will be described in detail with reference to FIG. 3.

At least a portion of the gas flow path 111 may be defined inside the main body 110, and one side of the main body 110 is opened. For example, the main body 110 may extend in the front and rear direction and a front surface thereof may be opened.

Opened one side of the main body 110 may be in communication with the gas flow path 111. In one example, the gas outlet 150 may be disposed on the main body 110 to shield the opened one side of the main body 110.

The opened one side of the main body 110 may correspond to an end of the gas flow path 111, and the end of the gas flow path 111 may correspond to the gas outlet 150. For example, the gas outlet 150 may be composed of the gas flow path 111 exposed through the open front surface of the main body 110 and an outer wall front end 112 of the main body 110.

In one example, as shown in FIG. 4, in an embodiment of the present disclosure, the gas outlet 150 may include a center portion 154 and side portion 156 through which the gas is discharged. The gas flowing along the gas flow path 111 may be simultaneously delivered to the center portion 154 and the side portion 156 to be discharged to the outside.

The center portion 154 and the side portion 156 may correspond to discharge holes through which the gas is discharged from the gas outlet 150. The center portion 154 may be defined at a central side on the cross-section of the gas outlet 150, and a cross-section shape thereof may be circular. However, a shape of the center portion 154 may be a polygonal shape such as a square and the like as needed, and a size of a diameter thereof may also be varied as needed.

The side portion 156 may be defined to surround the center portion 154. For example, as shown in FIG. 4, the center portion 154 may be defined in a substantially circular shape at the center of the gas outlet 150, and the side portion 156 may be an opening in a shape of a ring in which the center portion 154 is defined at a center thereof.

In the present disclosure, the ring shape may have an extended shape of a closed curve shape. For example, FIG. 4 discloses the side portion 156 having a circular ring shape.

The ring shape may not necessarily be circular, and may be, for example, a polygonal ring shape such as a triangle, a square, or the like. That is, in an embodiment of the present disclosure, the side portion 156 may be in the circular ring or the polygonal ring shape, and FIG. 4 shows the side portion 156 having a substantially circular ring shape.

Further, the center portion 154 and the side portion 156 may be in communication with the same gas flow path 111 together. Referring to FIG. 3, there is one gas flow path 111 extending from the handle 180 inside the main body 110. The center portion 154 and the side portion 156 of the gas outlet 150 are in communication with the gas flow path 111 together to discharge the gas at the same time.

The discharge gas discharged from the side portion 156 may form a sense of volume for an entirety of the discharge gas discharged through the gas outlet 150. That is, a cross-sectional area of the entirety of the discharge gas may correspond to a size of a closed cross-section formed by the side portion 156.

However, the discharge gas of the side portion 156 may be diffused while being flowed, and a portion of the gas flow may be distributed toward a center on the cross-section where the gas is not discharged by the side portion 156, and thus, the cross-sectional area of the discharge gas may be reduced.

Accordingly, in an embodiment of the present disclosure, the center portion 154 is defined at a center of the side portion 156, and the phenomenon in which the discharge gas of the side portion 156 is distributed toward the center on the cross-section is suppressed by discharge gas of the center portion 154.

That is, the discharge gas of the center portion 154 flows from the center on the cross-section of the entire discharge gas of the gas outlet 150, and suppresses the discharge gas of the side portion 156 from being distributed toward the center during the flow process, so that it may be advantageous for the entire discharge gas to maintain an initial cross-sectional area thereof.

Accordingly, discharge gas having a large cross-sectional area may be provided to the user, and the user may perform dry using the bulky gas. For example, the entire discharge gas with the volume formed through the center portion 154 and the side portion 156 may allow the user to perform the dry in a larger area.

Further, in an embodiment of the present disclosure, because the center portion 154 and the side portion 156 are in communication with one gas flow path 111, the gas flow paths 111 respectively for the center portion 154 and the side portion 156 may not separately defined. Thus, it may be advantageous in terms of design and may be efficient in providing three-dimensional discharge gas to the user.

In one example, referring to FIG. 4, in an embodiment of the present disclosure, the gas outlet 150 further includes a discharge base 152 disposed on the opened one side of the main body 110. The center portion 154 may be defined at a center of the discharge base 152, and the side portion 156 may be defined between an outer circumferential surface of the discharge base 152 and an outer wall of the main body 110.

FIG. 4 illustrates the discharge base 152 coupled to the opened one side of the main body 110. The discharge base 152 may be disposed to correspond to an opened cross-sectional shape of the one side of the main body 110, but may not be limited thereto and may be formed in various shapes or materials.

For example, the discharge base 152 may be disposed to be partially different from the shape of the opened front surface of the main body 110 to determine the shape of the side portion 156, and may be molded with a material the same as or different from a material of the outer wall of the main body 110.

The discharge base 152 may constitute an entirety or a portion of one surface of the main body 110, for example, the front surface of the main body 110 as shown in FIG. 4, so that the center portion 154 may be defined at the center of the discharge base 152 and the side portion 156 may be defined between the outer circumferential surface of the discharge base 152 and the outer wall of the main body 110.

The discharge base 152 may be coupled to an opening of the main body 110 in various schemes, such as a scheme using a plurality of coupling ribs, and may be integrally molded with the main body 110.

In one example, as shown in FIG. 4, in an embodiment of the present disclosure, the discharge base 152 may have a shape of being indented toward an interior of the main body 110 from the side portion 156 toward the center portion 154.

A center of a front surface of the discharge base 152 may be indented toward the interior of the main body 110, so that the front surface of the discharge base 152 may form a curved surface. Accordingly, the discharge gas of the center portion 154 on the flow path of the discharge gas discharged to the gas outlet 150 may be discharged upstream from the discharge gas of the side portion 156.

When the discharge gas of the center portion 154 on the flow path of the entire discharge gas starts to be diffused prior to the discharge gas of the side portion 156, the cross-section of the discharge gas of the central portion 154 may be increased through the diffusion, and an effect in which the discharge gas of the center portion 154 with an increased cross-sectional area suppresses the discharge gas of the side portion 156 from being flowed or discharged toward the center may be increased.

Further, the front surface of the discharge base 152 constituting a portion of a space in which the discharge gas of the center portion 154 is diffused forms the curved surface, so that it may be advantageous in preventing formation of unnecessary turbulence. A curvature of the curved surface formed by the front surface of the base portion may be variously set as necessary.

In one example, an embodiment of the present disclosure may further include a guide cone disposed at a center of the center portion 154 and guiding the flow of the gas discharged through the center portion 154. The gas may be discharged between an inner surface of the center portion 154 and the guide cone.

FIG. 4 illustrates the guide cone disposed at the center of the center portion 154. As the guide cone is disposed, the discharge gas of the center portion 154 is discharged into a space between the inner surface of the center portion 154 and an outer surface of the guide cone.

When the guide cone is disposed at the center of the center portion 154, the center portion 154 may correspond to a ring-shaped discharge hole. That is, the discharge gas of the center portion 154 may have a ring-shaped cross-section and may be discharged from the center portion 154.

As described above, the discharge gas of the center portion 154 may contribute to suppressing the reduction of the cross-sectional area resulted from the discharge gas of the side portion 156 that flows toward the center in the flow process. In addition, an embodiment of the present disclosure may increase a level at which the discharge gas of the center portion 154 diffuses outward from the cross-section by disposing the guide cone at the center of the center portion 154.

When the cross-sectional area of the discharge gas of the center portion 154 is increased as the guide cone is disposed, the effect of suppressing the phenomenon in which the discharge gas of the side portion 156 flows inward of the cross-section may be increased.

In one example, in the guide cone, a rear end protruding toward the gas flow path 111 and a front end protruding in a discharge direction of the gas of the center portion 154 may respectively have conical shapes. The conical shape means a shape in which a cross-section has a circular shape and a diameter of the circle gradually decreases as a length increases.

However, in the conical shape, the circular shape may include a shape other than a definite circular shape such as an ellipse and the like, and the reduction in the diameter may not necessarily be constant, for example, a diameter reduction rate may gradually increase or gradually decrease.

Further, as the front end of the guide cone protrudes in the conical shape, an effect in which the discharge gas of the center portion 154 is concentrated toward the rim of the center portion 154 increases. Thus, the effect of suppressing the discharge gas of the side portion 156 from flowing toward the discharge gas of the center portion 154 may be further increased.

An outer circumferential surface of the guide cone may have a shape corresponding to an inner circumferential surface of the center portion 154, and a separation distance between the outer circumferential surface of the guide cone and the inner circumferential surface of the center portion 154 may be varied as needed.

Further, the guide cone may be made of a material the same as or different from the material of the discharge base 152, and a curvature of the outer surface thereof may be variously designed as needed.

In one example, the gas outlet 150 may further include a discharge guide ring. The discharge guide ring may be disposed on the inner surface of the center portion 154 and protrude in the discharge direction of the gas of the center portion 154 to guide the gas flow together with the guide cone. FIG. 4 illustrates that the guide cone and the discharge guide ring are arranged in the center portion 154.

The discharge guide ring may have a ring shape extending along the rim of the center portion 154, and may be integrally molded with the discharge base 152 or molded separately from the discharge base 152 to be coupled to the inner circumferential surface of the center portion 154.

The discharge guide ring may protrude outward from the center portion 154 or the discharge base 152 based on the gas discharge direction. The flow of the discharge gas of the center portion 154 may be concentrated between the guide cone and the discharge guide ring by the guide cone and the discharge guide ring protruding from the center portion 154.

A protruding end of the discharge guide ring may have a curved shape to facilitate the gas flow. A diameter of the discharge guide ring may be different for each portion, and the shape thereof may also be varied as needed.

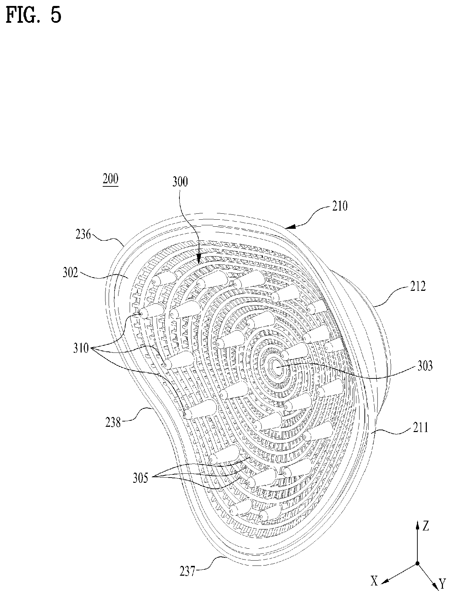

In one example, FIG. 5 shows the diffuser 200 according to an embodiment of the present disclosure. The diffuser 200 is removably coupled to the main body 110, so that the gas discharged from the gas outlet 150 is introduced into the diffuser 200 and the gas introduced into the diffuser 200 is discharged to the outside.

The hair dryer 100 according to an embodiment of the present disclosure may include the diffuser 200 as shown in FIG. 1, and the diffuser 200 may be removably coupled to the main body 110 of the hair dryer 100.

The diffuser 200 may be disposed such that gas discharged from the gas outlet 150 of the main body 110 flows into the diffuser 200. The diffuser 200 may be coupled to the main body 110 such that a rear side thereof covers the gas outlet 150, and the gas discharged from the gas outlet 150 may flow into the diffuser 200 through a gas inlet hole 215 defined at a rear side of the diffuser 200.

The user may selectively use the diffuser 200 for scalp or hair management. For example, the user may use the diffuser 200 including a massage protrusion 310 and a light irradiator 260, which will be described later, for scalp care, or may use the diffuser 200 at which a flow cross-sectional area of the gas is increased as needed in a hair drying step,
The rear side of the diffuser 200 may be coupled to the front end 112 of the main body 110. A first coupling portion 120 may be disposed at the front end 112 of the main body 110, and a second coupling portion 220 coupled to the first coupling portion 120 may be disposed at the rear side of the diffuser 200.

A coupling scheme between the diffuser 200 and the main body 110 may vary. The diffuser 200 may be coupled to the main body 110 in a scheme such as screw coupling, fitting coupling, magnetic coupling, sliding coupling, and the like to receive the gas from the main body 110.

An embodiment of the present disclosure may improve ease of use of the user as the diffuser 200 is disposed to be removable from the main body 110. For example, the user may use the hair dryer 100 by removing the diffuser 200 when the user is desired to use the gas discharged from the gas outlet 150 of the main body 110 as it is. Further, the user may use the hair dryer 100 coupled to the diffuser 200 when the user wants a more diffused flow cross-sectional area.

The diffuser 200 may include a diffusing case 210 and a discharge cover 300, and the diffusing case 210 and a discharge cover 300 may form an exterior of the diffuser 200.

An inner diameter of the diffuser 200 may increase the inner diameter in a forward direction. That is, the diffusing case 210 of the diffuser 200 may be disposed such that an internal cross-sectional area thereof viewed from the front increases from a rear side 212 to a front side 211.

Accordingly, the gas delivered from the gas outlet 150 may be provided to the user in a state in which the flow cross-sectional area thereof is increased as the gas speed is reduced in the forward direction of the diffuser 200. The user may use the diffuser 200 for natural drying, styling, and the like of the hair.

The front side 211 of the diffusing case 210 may be opened to define an open front surface. An entirety or a portion of the front surface of the diffuser 200, that is, the diffusing case 210, may define the open surface.

The gas present inside the diffuser 200 may be discharged to the outside through the open surface of the diffusing case 210. That is, the gas inside the diffuser 200 may be provided to the user while being discharged forward through the front side 211 of the diffusing case 210.

**In** the diffuser 200, the open surface defined in the front side 211 of the diffusing case 210 may be exposed to the outside as it is, or the discharge cover 300 may be provided coupled to the open surface.

FIG. 5 shows a state in which the open surface is present in the front side 211 of the diffusing case 210 according to an embodiment of the present disclosure and the discharge cover 300 is coupled to the open surface.

The discharge cover 300 coupled to the open surface defined in the front side 211 of the diffusing case 210 may include a gas discharge hole 305 defined therein through which the gas may be discharged. The discharge cover 300 has a shape corresponding to the open surface of the diffusing case 210 and may be coupled to the diffusing case 210 to be located on the open surface.

A plurality of gas discharge holes 305 may be defined and may be spaced apart from each other in the front surface of the discharge cover 300. FIG. 5 shows the discharge cover 300 in which the plurality of gas discharge holes 305 are uniformly distributed and arranged in the front surface.

Accordingly, in the diffuser, the gas may be discharged through the entirety of the front surface of the discharge cover 300 and the user may receive the gas that is discharged forward of the discharge cover 300 and uniformly dispersed.

The discharge cover 300 may be disposed such that an edge 302 located on the outermost side along a radial direction of the diffuser 200 is in close contact with the diffusing case 210. That is, the diffusing case 210 may have a front circumferential portion 236 surrounding the open surface in the front side 211, and the discharge cover 300 may be disposed such that the edge 302 has a shape corresponding to the front circumferential portion 236 and in contact with the front circumferential portion 236.

As shown in FIG. 5, the diffuser 200 according to an embodiment of the present disclosure may have a first portion 237 and a second portion 238 on the front circumferential portion 236 of the diffusing case 210. The first portion 237 and the second portion 238 may be arranged with different distances from the rear side 212 of the diffusing case 210, for example, the gas inlet hole 215 of the diffusing case 210.

In addition, the discharge cover 300 may be disposed such that the edge 302 is molded to correspond to shapes of the first portion 237 and the second portion 238 to be in close contact with the front circumferential portion 236 of the diffusing case 210.

In an embodiment of the present disclosure, the front circumferential portion 236 of the diffusing case 210 and the edge 302 of the discharge cover 300 may be designed to fit a head of the user with an arbitrary curved surface while respectively having curvatures and having different lengths protruding forward along an outer circumferential direction of the diffuser 200. Accordingly, a rate of adhesion with the scalp or the hair of the user may be efficiently increased to minimize a space between the head of the user and the diffuser 200.

Accordingly, an amount of the gas discharged forward of the discharge cover 300 or the light provided by the light irradiator 260 may be efficiently increased.

An ergonomic design is made through the front circumferential portion 236 of the diffusing case 210 and the edge 302 of the discharge cover 300 respectively arranged to form curves when viewed from the side as described above. In this case, the curvatures and the like of the front circumferential portion 236 and the edge 302 may be designed based on a standard head that is statistically determined.

For example, an embodiment of the present disclosure may define a R127 curvature design from a shape of the standard head, and design the shapes of the front circumferential portion 236 and the edge 302 to correspond thereto.

In one example, in an embodiment of the present disclosure, a proximity sensor 269 may be disposed inside the diffusing case 210 to improve ease of use and efficiency of the diffuser 200, and an open region 303 may be defined in the discharge cover 300 such that a distance measurement accuracy of the proximity sensor 269 for the target in front of the diffuser 200, for example, the hair or the scalp of the user may be improved.

That is, the proximity sensor 269 may be in various schemes such as pressure, ultrasound, infrared, and the like to measure the distance to the target in front of the proximity sensor 269, and a region of the discharge cover 300 in front of the proximity sensor 269 may be opened to define the open region 303.

In one example, FIG. 5 shows the discharge cover 300 on which a plurality of massage protrusions 310 are arranged. The massage protrusion 310 may have a pillar shape protruding forward of the diffuser 200 and may press the scalp of the user to provide a massage effect.

A cross-section shape, a protruding length, an arrangement form, and the like of the massage protrusion 310 may be variously determined in terms of a design. An embodiment of the present disclosure provides the user with scalp massage through the massage protrusion 310 while providing the gas diffused through a front surface of the discharge cover 300 to the user, thereby providing the improved ease of use and the scalp and hair care effects.

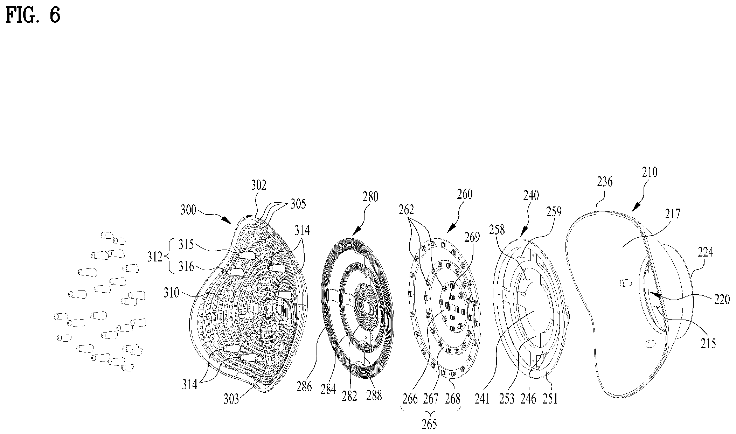

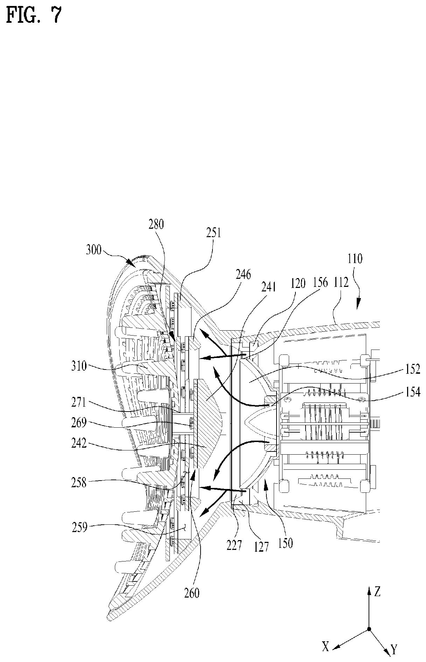

FIG. 6 is a view showing an exploded view of the diffuser 200 according to an embodiment of the present disclosure, and FIG. 7 is a view showing an internal cross-section of the diffuser 200 according to an embodiment of the present disclosure.

Referring to FIGS. 6 and 7, the diffuser 200 according to an embodiment of the present disclosure may include the diffusing case 210, a guide frame 240, the light irradiator 260, a light diffusion frame 280, and the discharge cover 300.

In the diffusing case 210, the rear side 212 may be coupled with the main body 110, and the open surface may be defined in the front side 211. The inner diameter of the diffusing case 210 may increase from the rear side 212 to the front side 211, so that the inside gas may be diffused and discharged to the outside.

That is, the gas discharged through the gas outlet 150 of the main body 110 may be provided to the user in a state in which the flow cross-sectional area thereof is increased as the gas is flowing in the diffusing case 210.

FIGS. 6 and 7 show the diffusing case 210 in which the inner diameter thereof increases from the rear side 212 to the front side 211 and accordingly an outer diameter thereof increases in the same manner.

The gas inlet hole 215 may be defined in the rear side 212 of the diffusing case 210. When the diffusing case 210 is coupled to the main body 110, the gas inlet hole 215 is positioned to face the gas outlet 150. Further, the gas discharged from the gas outlet 150 may be introduced into the diffusing case 210 through the gas inlet hole 215.

The gas inlet hole 215 may be located at a center of the rear side 212 of the diffusing case 210 when viewed from the rear, and a cross-section shape of the gas inlet hole 215 may correspond to the gas outlet 150. For example, the gas inlet hole 215 is defined to have an inner diameter larger than the side portion 156 of the gas outlet 150, so that the gas discharged from the gas outlet 150 may be completely introduced into the diffusing case 210 through the gas inlet hole 215.

The second coupling portion 220 coupled to the main body 110 may be disposed on the rear side 212 of the diffusing case 210. The diffusing case 210 may include a rear circumferential portion 217 surrounding the gas inlet hole 215 in the rear side 212, and the second coupling portion 220 may be disposed at the rear circumferential portion 217.

The second coupling portion 220 may further include a coupling sleeve 224. The coupling sleeve 224 may extend rearward from the rear circumferential portion 217. The coupling sleeve 224 may be disposed to outwardly surround the front end 112 of the main body 110.

The second coupling portion 220 may have a second magnetic fastening portion 227 embedded in the rear circumferential portion 217 or located inside the rear circumferential portion 217, and may include a power receiver disposed on an inner surface and the like of the coupling sleeve 224.

In addition, the first coupling portion 120 may be disposed at the front end 112 of the main body 110, may have a first magnetic fastening portion 127 embedded inside the outer wall of the front end 112 or located inside the outer wall, and may include a power transmitter disposed on an outer surface and the like of the outer wall of the front end 112.

The first coupling portion 120 is coupled to the second coupling portion 220. At least one of the first magnetic fastening portion 127 and the second magnetic fastening portion 227 may include a magnetic force generator, so that the first magnetic fastening portion 127 and the second magnetic fastening portion 227 may be magnetically coupled to each other. The magnetic coupling means a scheme of mutual coupling through a magnetic force generated from the magnetic force generator such as a magnet and an electromagnet.

The power transmitter may supply power to the power receiver in contact or connection with the power receiver. The power receiver may be connected to a component inside the diffuser 200, for example, the light irradiator 260 and the like to supply power thereto.

The open surface surrounded by the front circumferential portion 236 may be defined in the front side 211 of the diffusing case 210, and the gas inside the diffusing case 210 may be discharged forward of the diffuser 200 through the open surface in the front side 211.

In one example, the guide frame 240 may be disposed inside the diffusing case 210. The guide frame 240 is disposed to guide the flow of the gas introduced through the gas inlet hole 215.

The guide frame 240 may be disposed to face the gas inlet hole 215 of the diffusing case 210. The guide frame 240 may have a diffusion portion 241 at a center thereof, a first guide 246 disposed radially outward of the diffusion portion 241, and a second guide 251 disposed radially outward of the first guide 246.

The guide frame 240 may include a guide connector 253 extending along the radial direction of the diffuser 200, and the guide connector 253 may connect the diffusion portion 241, the first guide 246, and the second guide 251 to each other.

The diffusion portion 241 of the guide frame 240 is disposed to face the gas inlet hole 215 to diffuse the gas introduced through the gas inlet hole 215 outward in the radial direction. That is, the flow cross-sectional area of the gas introduced through the gas inlet hole 215 may be increased by the diffusion portion 241.

According to an embodiment of the present disclosure, in the gas outlet 150 having the center portion 154 and the side portion 156, a flow direction of the gas discharged from the center portion 154 may be changed by the diffusion portion 241. That is, the diffusion portion 241 may have a larger diameter than the center portion 154 and diffuse the gas provided from the center portion 154 outward in the radial direction.

The first guide 246 may have a ring shape, and the diffusion portion 241 may be located at a center of the first guide 246. The diffusion portion 241 may have a circular cross-section, and may be outwardly spaced apart from the diffusion portion 241 while being concentric with the diffusion portion 241 of the first guide 246.

A first flow path 258 may be located between the first guide 246 and the diffusion portion 241. That is, the first guide 246 may be spaced apart from the diffusion portion 241 to define the first flow path 258 between the first guide 246 and the diffusion portion 241. The gas diffused through the diffusion portion 241 may flow through the first flow path 258.

The second guide 251 may have a ring shape corresponding to the first guide 246, and the diffusion portion 241 and the first guide 246 may be located at a center of the second guide 251. That is, the second guide 251 may be concentric with the diffusion portion 241 and the first guide 246 and may be spaced apart from the first guide 246.

That is, an inner diameter of the first guide 246 may be larger than the diameter of the diffusion portion 241, and an inner diameter of the second guide 251 may be larger than an outer diameter of the first guide 246. Accordingly, the first flow path 258 may be defined between the diffusion portion 241 and the first guide 246, and a second flow path 259 may be defined between the first guide 246 and the second guide 251.

The gas diffused by the diffusion portion 241 may flow through the first flow path 258 and the second flow path 259. An outer diameter of the second flow path 259 may be larger than the diameter of the gas inlet hole 215, so that the gas introduced through the gas inlet hole 215 may be diffused by the diffusion portion 241 and flow with a larger flow cross-section.

The light irradiator 260 may be located in front of the guide frame 240. The light irradiator 260 may be installed on a front surface of the guide frame 240. The light irradiator 260 may have a plurality of light emitters 262 arranged on a circuit board 265. The circuit board 265 may include a plurality of circuit boards separated from each other, and the plurality of boards may be respectively arranged on the diffusion portion 241, the first guide 246, and the second guide 251 of the guide frame 240.

The light irradiator 260 may include the plurality of circuit boards 265, and the plurality of circuit boards 265 may respectively include a central board 266, a first board 267, and a second board 268.

The central board 266 may have a cross-section shape corresponding to the diffusion portion 241. For example, the diffusion portion 241 may have the circular cross-section, and the central board 266 may have a circular cross-section in the same manner as the diffusion portion 241, may be disposed on a front surface of the diffusion portion 241, and may include the plurality of light emitters 262.

The first board 267 may have a shape corresponding to the first guide 246. For example, the first guide 246 may have the ring shape, and the first board 267 may have a ring shape in the same manner as the first guide 246, may be disposed on a front surface of the first guide 246, and may include the plurality of light emitters 262.

The second board 268 may have a shape corresponding to the second guide 251. For example, the second guide 251 may have the ring shape, and the second board 268 may have a ring shape in the same manner as the second guide 251, may be disposed on a front surface of the second guide 251, and may include the plurality of light emitters 262.

The central board 266, the first board 267, and the second board 268 may be arranged to be concentric like the guide frame 240. The first board 267 may be outwardly spaced apart from the central board 266, and the second board 268 may be outwardly spaced apart from the first board 267.

That is, an inner diameter of the first board 267 may be larger than a diameter of the central board 266, and an inner diameter of the second board 268 may be larger than an outer diameter of the first board 267. Therefore, like the guide frame 240, the first flow path 258 may be located between the central board 266 and the first board 267, and the second flow path 259 may be located between the first board 267 and the second board 268.

The light irradiator 260 may irradiate light toward the front side 211 of the diffusing case 210 through the plurality of light emitters 262. The light irradiated from the light irradiator 260 may be provided to a location ahead of the diffuser 200 through the front side 211 of the diffusing case 210.

For example, the light irradiated from the light irradiator 260 may be provided to the location ahead of the diffuser 200 by passing through the open surface of the diffusing case 210, passing through the gas discharge hole 305 of the discharge cover 300, passing through the discharge cover 300, or passing through the massage protrusion 310 of the discharge cover 300.

As the light is irradiated to the location ahead of the diffuser 200, the diffuser 200 according to an embodiment of the present disclosure may perform user's hair or scalp care. The light irradiated from the light irradiator 260 may contribute to improving scalp and hair health while drying the user's scalp or hair or providing heat to the user's scalp or hair.

The proximity sensor 269 may be disposed on the circuit board 265 of the light irradiator 260. FIG. 6 shows a state in which the proximity sensor 269 is disposed on the central board 266 of the light irradiator 260 according to an embodiment of the present disclosure.

The proximity sensor 269 may be disposed at a center of the central board 266. The proximity sensor 269 may be disposed to measure the separation distance from the target positioned in front of the proximity sensor 269. The controller 115 may be disposed to control the light irradiator 260 based on the separation distance between the proximity sensor 269 and the front target measured by the proximity sensor 269.

For example, when the separation distance from the target measured by the proximity sensor 269 is equal to or less than a reference distance, the controller 115 may control the light irradiator 260 such that the light irradiator 260 irradiates the light forward. The reference distance may be predetermined in terms of a design or control.

However, the light irradiator 260 may be operated through a physical switch that is operated when the separation distance measured by the proximity sensor 269 is equal to or less than the reference distance.

**In** an embodiment of the present disclosure, as the proximity sensor 269 is used, the light irradiator 260 is operated when the separation distance from the target in front of the diffuser 200, for example, the scalp or the hair of the user is equal to or less than the reference distance, thereby improving ease of use and an operation efficiency.

The proximity sensor 269 may be disposed in various types. For example, the proximity sensor 269 may be a pressure sensor that detects whether a pressing force is applied from the user's scalp or hair, or a photosensitive sensor that measures a level at which an amount of sensed light decreases as the separation distance from the scalp or the hair decreases.

**In** addition, the proximity sensor 269 may be an **IR** sensor that measures an infrared ray transmitted from the front target, that is, the user's scalp or hair to measure the separation distance from the scalp or the hair. **In** this case, the proximity sensor 269 may be disposed to irradiate the infrared ray forward.

**In** one example, the light diffusion frame 280 may be located in front of the light irradiator 260. The light diffusion frame 280 may be installed on a front surface of the light irradiator 260. That is, the light diffusion frame 280 may be disposed to forwardly cover the light irradiator 260.

The light diffusion frame 280 may include a central light diffusion portion 282, a first light diffusion portion 284 and a second light diffusion portion 286. The light diffusion frame 280 may further include a light diffusion connector 288 for connecting the central light diffusion portion 282, the first light diffusion portion 284, and the second light diffusion portion 286 to each other.

The central light diffusion portion 282 may have a cross-section shape corresponding to the central board 266. For example, the central board 266 may have the circular cross-section, and the central light diffusion portion 282 may have a circular cross-section in the same manner as the central board 266 and may cover the front surface of the diffusion portion 241.

The first light diffusion portion 284 may have a shape corresponding to the first board 267. For example, the first board 267 may have the ring shape, and the first light diffusion portion 284 may have a ring shape in the same manner as the first board 267 and may cover the front surface of the first board 267.

The second light diffusion portion 286 may have a shape corresponding to the second board 268. For example, the second board 268 may have the ring shape, and the second light diffusion portion 286 may have a ring shape in the same manner as the second board 268 and may cover the front surface of the second board 268.

The central light diffusion portion 282, the first light diffusion portion 284, and the second light diffusion portion 286 may be arranged to be concentric like the guide frame 240 and the light irradiator 260. The first light diffusion portion 284 may be outwardly spaced apart from the central light diffusion portion 282, and the second light diffusion portion 286 may be outwardly spaced apart from the first light diffusion portion 284.

That is, an inner diameter of the first light diffusion portion 284 may be larger than a diameter of the central light diffusion portion 282, and an inner diameter of the second light diffusion portion 286 may be larger than an outer diameter of the first light diffusion portion 284. Accordingly, like the guide frame 240, the first flow path 258 may be located between the central light diffusion portion 282 and the first light diffusion portion 284, and the second flow path 259 may be located between the first light diffusion portion 284 and the second light diffusion portion 286.

That is, the diffuser 200 according to an embodiment of the present disclosure may be disposed in a shape in which the first flow path 258 and the second flow path 259 are extended in the front and rear direction through the guide frame 240, the light irradiator 260, and the light diffusion frame 280.

The light diffusion connector 288 may be disposed in a shape corresponding to the guide connector 253. For example, the guide connector 253 and the light diffusion connector 288 may have an extended shape along the radial direction of the diffuser 200.

The light diffusion connector 288 may be located in front of the guide connector 253. The light diffusion frame 280 may be fixed inside the diffusing case 210 as the light diffusion frame 280 is fastened to the guide connector 253.

An embodiment of the present disclosure is advantageous in terms of a design and structurally stable in that, in a state in which the guide frame 240 is constituted by a plurality of components, the plurality of components are able to be handled as a single component through the guide connector 253.

In addition, an embodiment of the present disclosure is advantageous in terms of the design and structurally stable in that, in a state in which the light diffusion frame 280 is constituted by a plurality of components, the plurality of components are able to be handled as a single component through the light diffusion connector 288.

Furthermore, the light diffusion connector 288 of the light diffusion frame 280 is coupled to the guide connector 253 of the guide frame 240, so that all of the central light diffusion portion 282, the first light diffusion portion 284, and the second light diffusion portion 286 may be stably fixed, which is advantageous in terms of coupling.

The light diffusion frame 280 may be made of a material through which light is transmitted. For example, the light diffusion frame 280 may be made of a transparent or translucent material. The light irradiated from the light irradiator 260 may be scattered and diffused while passing through the light diffusion frame 280.

In the diffuser 200 according to an embodiment of the present disclosure, the light diffusion frame 280 is disposed in front of the light irradiator 260, so that the light irradiated from the light irradiator 260 may be provided to the user while being scattered and diffused and being uniformly dispersed in a larger area.

A treatment for the diffusion or the scattering of the light may be performed on a front surface or a rear surface of the light diffusion frame 280. For example, etching may be performed or a pattern through laser processing and the like may be formed on a surface of the light diffusion frame 280.

**In** one example, the central light diffusion portion 282 is disposed to shield the front surface of the central board 266, and a portion of the central light diffusion portion 282 in front of the proximity sensor 269 may be opened such that the measurement of the separation distance from the target in front of the diffuser 200 by the proximity sensor 269 disposed on the central board 266 is easy.

FIG. 6 shows a state in which the proximity sensor 269 is disposed at the center of the central board 266 according to an embodiment of the present disclosure and the central light diffusion portion 282 has a hole defined at a center thereof to expose the proximity sensor 269 forwardly.

The discharge cover 300 may be disposed to shield the open surface defined in the front side 211 of the diffusing case 210 in which the guide frame 240, the light irradiator 260, and the light diffusion frame 280 are embedded. The plurality of gas discharge holes 305 are defined in the discharge cover 300, so that the gas may be discharged and the light may be irradiated forward.

The discharge cover 300 may be disposed such that the edge 302 has a curvature to correspond to the front circumferential portion 236 of the diffusing case 210 when viewed from the side and is indented rearwards centerwardly when viewed from the front.

That is, a front surface of the discharge cover 300 may form a curved surface that is indented rearwards centerwardly, so that the discharge cover 300 may have a shape corresponding to the head of the user and may be optimized to provide the massage effect through the massage protrusion 310 while providing the gas and the light to the user.

The plurality of massage protrusions 310 protruding or extending forward on the front surface of the discharge cover 300 may be arranged, and a contact portion may be disposed on the surface of the discharge cover 300 such that a sense of touch with the scalp or the hair of the user may be improved and damage to the scalp and the hair may be minimized. The contact portion may be made of a material such as silicon and the like.

Some of the plurality of massage protrusions 310 may correspond to the moisture measurement protrusions 312. That is, in the diffuser 200 according to an embodiment of the present disclosure, the plurality of massage protrusions 310 may include the moisture measurement protrusions 312.

The moisture measurement protrusion 312 may be disposed to measure a moisture amount of the scalp or the hair of the user. A pair of the moisture measurement protrusions 312 may be arranged to measure an impedance, that is, bioelectrical impedance through an electric field formed therebetween.

The moisture measurement protrusion 312 may be connected to the controller 115. Further, the controller 115 may determine the impedance using a voltage, a current, a resistance, and the like, which are identified through the moisture measurement protrusion 312, determine the moisture amount of the scalp or the hair of the user based on the determined impedance, and control an operation of the fan 119, the temperature adjuster 117, or the light irradiator 260 based on the determined moisture amount.

For example, the controller 115 may control the fan 119 such that the gas speed increases as the moisture amount of the scalp or the hair of the user increases, control the temperature adjuster 117 such that the gas temperature increases, or control the light irradiator 260 such that the light amount increases.

The pair of moisture measurement protrusions 312 may be arranged. The moisture measurement protrusions 312 may include a first moisture measurement protrusion 315 electrically having a first pole and a second moisture measurement protrusion 316 having a second pole opposite to the first pole.

The controller 115 may determine the impedance and the moisture amount through the electric field formed between the first moisture measurement protrusion 315 and the second moisture measurement protrusion 316.

A plurality of pairs of moisture measurement protrusions 312, each of which is constituted by the first moisture measurement protrusion 315 and the second moisture measurement protrusion 316, may be arranged. One pair of moisture measurement protrusions 312 may be disposed to be spaced apart from another pair of moisture measurement protrusions, and different massage protrusions 310 may be positioned therebetween.

In one example, the open region 303 may be defined at the center of the discharge cover 300. The proximity sensor 269 may be exposed forward through the hole defined in the light diffusion frame 280 and the open region 303 of the discharge cover 300, and may wholly measure the separation distance from the target in front of the diffuser 200. A protection member that protects the proximity sensor 269 and allows the infrared ray or the like to pass straight therethrough may be disposed in front of the proximity sensor 269.

FIG. 7 shows an internal cross-section of the diffuser 200 and a state in which the diffuser 200 is coupled to the main body 110 to receive the gas from the gas outlet 150.

Referring to FIG. 7, in an embodiment of the present disclosure, the first coupling portion 120 of the main body 110 may include the first magnetic fastening portion 127 and the second coupling portion 220 of the diffuser 200 may include the second magnetic fastening portion 227.

The diffuser 200 may be coupled to the front end 112 of the main body 110 through the magnetic coupling between the first magnetic fastening portion 127 and the second magnetic fastening portion 227. The first coupling portion 120 may further include a hook fastener and the second coupling portion 220 may further include a hook fastened to the hook fastener, so that a coupling stability between the diffuser 200 and the main body 110 may be enhanced.

Hereinafter, the flow of the gas discharged from the gas outlet 150 according to an embodiment of the present disclosure will be described with reference to FIG. 7.

In the gas outlet 150, the gas is discharged from the center portion 154 and the side portion 156. The gas inlet hole 215 of the diffusing case 210 is defined to have a diameter equal to or larger than that of the side portion 156 and face the gas outlet 150, so that the gas discharged from the center portion 154 and the side portion 156 may be introduced into the inlet hole 215.

The guide frame 240 may be disposed inside the diffusing case 210 to face the gas outlet 150. Specifically, the diffusion portion 241 of the guide frame 240 may be positioned to face the center portion 154 of the gas outlet 150.

The gas discharged from the center portion 154 flows toward the diffusion portion 241. As the diffusion portion 241 has a diameter larger than that of the center portion 154, the gas discharged from the center portion 154 may be diffused along the radial direction of the diffuser 200.

The diffusion portion 241 may have a diffusion protrusion 242 on a rear surface thereof facing the center portion 154, and the diffusion effect of the gas discharged from the center portion 154 may be improved by the diffusion protrusion 242. The diffusion protrusion 242 may be disposed to increase in rearwardly protruding height toward a center on the cross-section when viewed from the rear.

At least a portion of the gas discharged from the center portion 154 may flow along the first flow path 258 defined between the diffusion portion 241 and the first guide 246 in the guide frame 240 by the diffusion portion 241 and the diffusion protrusion 242.

In one example, the gas discharged from the side portion 156 may have a flow form outwardly surrounding the gas discharged from the center portion 154, and the gas discharged from the side portion 156 may also diffuse outward along the radial direction of the diffuser 200 as the gas of the center portion 154 is diffused by the diffusion portion 241.

Therefore, at least a portion of the gas discharged from the side portion 156 and at least a portion of the gas discharged from the center portion 154 may flow along the second flow path 259 defined between the first guide 246 and the second guide 251 in the guide frame 240.

**In** an embodiment of the present disclosure, despite a design feature that the inner diameter of the diffuser 200 increases forwardly, the discharging of the gas discharged from the center portion 154 and the side portion 156 in the forward direction while being maintained in a specific form may be effectively suppressed through the guide frame 240.

Furthermore, in an embodiment of the present disclosure, the diffuser 200 allows the gas discharged from the center portion 154 and the side portion 156 to be effectively dispersed and diffused with a larger flow cross-sectional area while preventing the flow of the gas from being maintained in the specific form.

In one example, the light irradiator 260 and the light diffusion frame 280 may be arranged in front of the guide frame 240 inside the diffusing case 210. The light irradiator 260 and the light diffusion frame 280 may be coupled with the guide frame 240 and thus may be handled as a single component, so that a structure that is excellent in a space utilization and is advantageous in design may be implemented.

In addition, the light irradiator 260 and the light diffusion frame 280 may define the first flow path 258 and the second flow path 259 together with the guide frame 240. In an embodiment of the present disclosure, as the structure in which the light irradiator 260 and the light diffusion frame 280 define the first flow path 258 and the second flow path 259 together with the guide frame 240 is achieved, the flow of the gas formed by the guide frame 240 may be effectively maintained and the gas may be discharged forwardly of the diffuser 200 through the light irradiator 260 and the light diffusion frame 280.

**In** one example, in the light irradiator 260, the first board 267 may be positioned forwardly of the central board 266, and the second board 268 may be positioned forwardly of the first board 267. Accordingly, the plurality of light emitters 262 arranged in the light irradiator 260 may be arranged to form a spherical surface that is substantially indented rearward.

Accordingly, the plurality of light emitters 262 may be arranged in a form in which a distance from a center of the light irradiator 260 along the radial direction increases forwardly. Such arrangement of the light emitters 262 may correspond to the shape of the front surface of the discharge cover 300 indented rearward.

That is, in an embodiment of the present disclosure, the plurality of light emitters 262 arranged on the light irradiator 260 are arranged to form the curved surface to correspond to the user's head having the curvature, so that a uniform amount of light may be provided to the user's scalp and hair.

Like the light irradiator 260, the guide frame 240 may be disposed such that the first guide 246 is positioned forwardly of the diffusion portion 241 and the second guide 251 is positioned forwardly of the first guide 246.

Accordingly, the first board 267 disposed on the front surface of the first guide 246 may be positioned forwardly of the central board 266 disposed on the front surface of the diffusion portion 241, and the second board 268 disposed on the front surface of the second guide 251 may be positioned forwardly of the first board 267.

Like the light irradiator 260, in the light diffusion frame 280, the first light diffusion portion 284 may be positioned forwardly of the central light diffusion portion 282 and the second light diffusion portion 286 may be positioned forwardly of the first light diffusion portion 284. Accordingly, a distance between the light diffusion frame 280 and the light irradiator 260 may be kept constant, and uniform dispersion and scattering of the light may be induced.

**In** addition, in the guide frame 240, as the second guide 251 is positioned forwardly of the first guide 246 and the first guide 246 is positioned forwardly of the diffusion portion 241, a space in which the gas introduced from the gas inlet hole 215 is diffused in the radial direction may be secured and the gas may be smoothly introduced into the first flow path 258 and the second flow path 259.

FIG. 7 shows the guide frame 240, the light irradiator 260, and the light diffusion frame 280 protruding forward in a direction away from centers thereof, according to an embodiment of the present disclosure.

**In** one example, FIG. 7 shows a light blocking portion 271 surrounding the proximity sensor 269. The light blocking portion 271 may be disposed to surround the proximity sensor 269 along the circumferential direction of the diffuser 200, thereby preventing a situation in which the light emitter 262 around the proximity sensor 269 affects the proximity sensor 269.

**In** addition, the light blocking portion 271 may be opened forward to prevent structural interference from occurring in a measurement of the separation distance between the diffuser 200 and the front target by the proximity sensor 269. For example, when the proximity sensor 269 measures the infrared ray transmitted from the target, the light blocking portion 271 is opened forward to allow the infrared ray transmitted from the target to be completely provided to the proximity sensor 269.

The light blocking portion 271 may be formed in a hollow cylindrical shape. The proximity sensor 269 may be located inside the light blocking portion 271. The light blocking portion 271 may have a shape extending from the central board 266 to the discharge cover 300.

The light blocking portion 271 may be disposed to extend rearward from the discharge cover 300, or may be formed integrally with the discharge cover 300 or integrally with the central board 266. The light blocking portion 271 may be manufactured separately from the discharge cover 300 and the central board 266, and may be coupled or connected to the discharge cover 300 and/or the central board 266.

In one example, as described above, the hair dryer 100 according to an embodiment of the present disclosure includes the main body 110, the handle 180, and the diffuser 200, and the diffuser 200 includes the diffusing case 210 and the discharge cover 300.

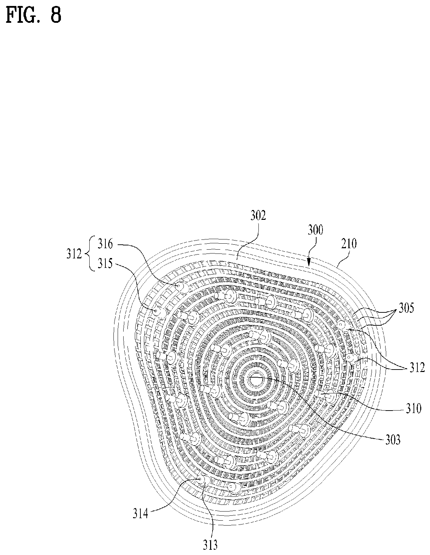

FIG. 8 shows a view of the diffuser 200 according to an embodiment of the present disclosure viewed from the front. Referring to FIG. 8, in an embodiment of the present disclosure, the discharge cover 300 may include the plurality of massage protrusions 310.

The plurality of massage protrusions 310 may protrude forward to press the target located in front of the discharge cover 300. In addition, the plurality of massage protrusions 310 may include the moisture measurement protrusion 312. The moisture measurement protrusion 312 may be disposed to measure the moisture amount of the target.

Specifically, in an embodiment of the present disclosure, the massage protrusion 310 may be disposed on the discharge cover 300, and may include the plurality of massage protrusions to press the front target, for example, the scalp, the hair, or the like of the user.

The massage protrusion 310 may have a shape of a protrusion protruding forward from the discharge cover 300, and a shape of a cross-section thereof may be various, such as circular or polygonal. A protrusion length of the massage protrusion 310 may vary in design.

For example, the plurality of massage protrusions 310 arranged on the discharge cover 300 may have the same protrusion length in an entire range of the discharge cover 300. Alternatively, a massage protrusion 310 disposed in a specific region may have a larger protrusion length than a massage protrusion 310 disposed in a remaining region.

The massage protrusion 310 may further include a contact cover forming a surface of the massage protrusion 310 to minimize damage to the user's scalp and hair. The contact cover may be made of a material, such as silicone, that may minimize the damage to the scalp and hair due to friction and the like.

FIG. 8 shows the plurality of massage protrusions 310 that are uniformly distributed throughout the front surface of the discharge cover 300 according to an embodiment of the present disclosure.

In one example, some of the plurality of massage protrusions 310 may correspond to the moisture measurement protrusions 312. That is, the plurality of massage protrusions 310 may include the moisture measurement protrusions 312. The moisture measurement protrusion 312 may be disposed to measure the moisture amount of the target.

The moisture measurement protrusions 312 corresponding to some of the plurality of massage protrusions 310 are arranged to press the user's scalp and the like in the same manner as the others of the massage protrusions 310. Furthermore, the moisture measurement protrusion 312 may be disposed to measure the amount of moisture present in the scalp or the like.

A moisture measurement scheme of the moisture measurement protrusion 312 may be various. For example, the moisture measurement protrusion 312 may be disposed to form the electric field to measure the bioelectrical impedance. A measurement scheme of the bioelectrical impedance in the present disclosure is as follows.

When the moisture measurement protrusion 312 measures the moisture amount of the scalp, a voltage may be formed at the moisture measurement protrusion 312 and an electric field may be formed by electrical polarity generation resulted from the voltage.

The electric field is changed by the amount of moisture present in the scalp. For example, as the moisture amount of the scalp increases, the electric field is amplified. Accordingly, an impedance value determined from the electric field may be reduced.

Conversely, when the moisture amount of the scalp is small, the electric field formed by the moisture measurement protrusion 312 is reduced, and thus the impedance value on the scalp may be measured as a large value.

As described above, in an embodiment of the present disclosure, the moisture measurement protrusion 312 may be disposed to measure the moisture amount of the scalp based on the change in the bioelectrical impedance, that is, the impedance defined in the present disclosure.

However, the moisture amount measurement scheme of the moisture measurement protrusion 312 according to an embodiment of the present disclosure may not be necessarily limited as described above, and may include various schemes such as an electro-osmosis scheme, an electronic sensor scheme, or the like.

The diffuser 200 according to an embodiment of the present disclosure may efficiently measure the moisture amount of the user's scalp or hair as some of the plurality of massage protrusions 310 are arranged as the moisture measurement protrusions 312.

Furthermore, the hair dryer 100 according to an embodiment of the present disclosure is advantageous because a result of the moisture amount measurement of the diffuser 200 may be used in the care of the user's scalp and hair.

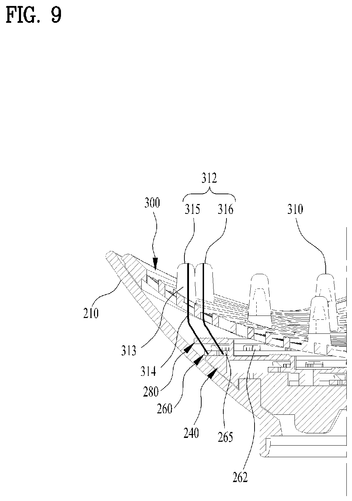

In one example, FIG. 9 shows the moisture measurement protrusion 312 connected to the light irradiator 260 according to an embodiment of the present disclosure.

Referring to FIGS. 8 and 9, in an embodiment of the present disclosure, the moisture measurement protrusion 312 may include a protrusion base 313 and a moisture measurement electrode 314.

The protrusion base 313 may be disposed to protrude forward to press the target. The moisture measurement electrode 314 may be disposed in the protrusion base 313, at least a portion of the moisture measurement electrode 314 may be exposed to the outside from the protrusion base 313, and the moisture measurement electrode 314 may have an electrical polarity by the voltage.

Specifically, the protrusion base 313 may have a shape of an protrusion protruding forward from the front surface of the discharge cover 300, and the protrusion base 313 may be included not only in the moisture measurement protrusion 312, but also in the others of massage protrusions 310.

For example, the moisture measurement protrusion 312 may be structurally the same as or similar to the others of the massage protrusions 310 except for the moisture measurement electrode 314.

The protrusion base 313 may protrude forward of the discharge cover 300 such that a front end thereof may press the user's scalp. The user may massage the scalp using the protrusion base 313 of the massage protrusion 310.

**In** one example, the moisture measurement electrode 314 may contain a conductive material such as copper and the like. Because the moisture measurement electrode 314 is made of the conductive material, the moisture measurement electrode 314 may be electrically, for example, positively or negatively charged when the voltage is provided.

The moisture measurement electrode 314 may be disposed in the protrusion base 313. At least the portion of the moisture measurement electrode 314 may be exposed to the outside from the protrusion base 313, and may be adjacent to or in contact with the scalp and the like of the user.

A portion of the moisture measurement electrode 314 may be located inside the protrusion base 313 or an entirety of the moisture measurement electrode 314 may be located on a surface of the protrusion base 313.

FIGS. 8 and 9 show the moisture measurement protrusion 312 having the moisture measurement electrode 314 exposed forward of the protrusion base 313 according to an embodiment of the present disclosure.

At the moisture measurement protrusion 312, the electrical polarity is generated at a portion of the moisture measurement electrode 314 exposed to the outside to generate the electric field on the scalp of the user. The electrical polarity may be either positive or negative.

In addition, in an embodiment of the present disclosure, the moisture measurement electrode 314 may extend to penetrate the protrusion base 313 and be exposed to the outside through an end of the protrusion base 313.

FIG. 9 shows the moisture measurement electrode 314 penetrating the protrusion base 313 along a longitudinal direction of the protrusion base 313 according to an embodiment of the present disclosure.

The moisture measurement electrode 314 may be made of the conductive material such as the copper and the like, and damage of the moisture measurement electrode 314 resulted from external contact and the like may easily occur. Therefore, in an embodiment of the present disclosure, the moisture measurement electrode 314 may be disposed inside the protrusion base 313.

The moisture measurement electrode 314 may be extend along the longitudinal direction of the protrusion base 313 to penetrate the interior of the protrusion base 313. The moisture measurement electrode 314 penetrating the protrusion base 313 may be exposed to the outside through a protruding end of the protrusion base 313.

As the protruding end of the protrusion base 313 is able to be in contact with the user's scalp or the like and the moisture measurement electrode 314 is exposed from the end of the protrusion base 313 to the outside, the moisture measurement electrode 314 may be in contact with or located adjacent to the user's scalp, which may be advantageous to form the electric field.

In addition, as the moisture measurement electrode 314 is disposed inside the protrusion base 313, damage or cutting of the moisture measurement electrode 314 due to contact may be effectively prevented.

In one example, in an embodiment of the present disclosure, the moisture measurement protrusion 312 may include the plurality of moisture measurement protrusions. In addition, the plurality of moisture measurement protrusions 312 may include a first moisture measurement protrusion 315 and a second moisture measurement protrusion 316.

The first moisture measurement protrusion 315 may include a moisture measurement electrode 314 having a first pole, and the second moisture measurement protrusion 316 may include a moisture measurement electrode 314 having a second pole opposite to the first pole. In the plurality of massage protrusions 310, the first moisture measurement protrusion 315 and the second moisture measurement protrusion 316 may be arranged adjacent to each other.

FIG. 8 shows the first moisture measurement protrusion 315 and the second moisture measurement protrusion 316 arranged to be adjacent to each other among the plurality of massage protrusions 310. The first moisture measurement protrusion 315 and the second moisture measurement protrusion 316 may be difficult to be distinguished from each other in terms of the exterior, but may be distinguished from each other with a difference in the electrical polarity.

For example, the first moisture measurement protrusion 315 may include the moisture measurement electrode 314 charged to have the first pole. The first pole may be an anode or a cathode.

The second moisture measurement protrusion 316 may include the moisture measurement electrode 314 charged to have the second pole. The second pole corresponds to the polarity opposite to the first pole. For example, when the first pole is the anode, the second pole may be the cathode, and when the first pole is the cathode, the second pole may be the anode.

As described above, the first moisture measurement protrusions 315 and the second moisture measurement protrusions 316 having the opposite electrical polarities may form an electric field by interaction. That is, the electric field may be formed between the first moisture measurement protrusion 315 and the second moisture measurement protrusion 316.

In an embodiment of the present disclosure, the first moisture measurement protrusion 315 and the second moisture measurement protrusion 316 having the different polarities are separated from each other, so that the moisture measurement electrode 314 of the first pole and the moisture measurement electrode of the second pole 314 of the second pole may be effectively and stably separated from each other.

In addition, because the first moisture measurement protrusion 315 and the second moisture measurement protrusion 316 are arranged to be adjacent to each other, the electric field by the first moisture measurement protrusion 315 and the second moisture measurement protrusion 316 may be easily generated.

In one example, FIG. 8 shows a state in which a plurality of pairs of moisture measurement protrusions 312 are arranged, wherein each pair of moisture measurement protrusions 312 is constituted by the first moisture measurement protrusion 315 and the second moisture measurement protrusion 316 are arranged.

Referring to FIG. 8, in the diffuser 200 according to an embodiment of the present disclosure, the plurality of massage protrusions 310 may include the plurality of pairs of moisture measurement protrusions 312, wherein each pair is constituted by the first moisture measurement protrusion 315 and the second moisture measurement protrusion 316.

The massage protrusions 310 may be spaced apart from each other over an entire area of the discharge cover 300, and the scalp or the hair of the user may have a different moisture amount for each portion thereof. For example, even when the moisture amount is relatively high in a specific region, the moisture amount may be low overall in the remaining regions other than the specific region.

That is, the scalp and the hair of the user may have the different moisture amount for each portion thereof. Accordingly, even when the moisture amount measured by the moisture measurement protrusion 312 in the specific region is high, the user's scalp or hair may be relatively dry overall.

When the moisture amount measured by the pair of moisture measurement protrusions 312 constituted by the first moisture measurement protrusion 315 and the second moisture measurement protrusion 316 is different from the overall moisture amount of the scalp and hair of the user as described above, a control result by the controller 115 capable of controlling multiple components based on the moisture amount may be different from a result required by the user.

Therefore, in an embodiment of the present disclosure, the plurality of pairs of moisture measurement protrusions 312 are arranged to measure the moisture amounts in different portions. That is, the plurality of pairs of moisture measurement protrusions 312 may be arranged to be spaced apart from each other.

That is, at least one general massage protrusion 310 other than the moisture measurement protrusion 312 may be disposed between one pair of moisture measurement protrusions 312 and another pair of moisture measurement protrusions 312.

FIG. 8 shows that a front surface region of the discharge cover 300 is divided into three regions, and the pair of moisture measurement protrusions 312 are disposed in each region. That is, in FIG. 8, three pairs of moisture measurement protrusions 312 are arranged. The three pairs of moisture measurement protrusions 312 may be spaced apart from each other to measure moisture amounts in different regions.

The number of pairs of moisture measurement protrusions 312 may be varied as necessary. **In** some cases, the moisture measurement protrusions 312 may be densely arranged in a specific region, or may be evenly distributed at equal spacings.

An embodiment of the present disclosure may utilize an average value of the moisture amounts respectively measured by the pairs of moisture measurement protrusions 312, or differently set importance of the regions to determine a comprehensive moisture amount and utilize the comprehensive moisture amount.

That is, the controller 115 may control the light irradiator 260 or the like using the average value of the moisture amounts respectively measured by the pairs of moisture measurement protrusions 312, or may control the light irradiator 260 or the like using a value reflecting moisture amounts of the remaining moisture measurement protrusions 312 in a predetermined manner to a moisture amount of a pair of measurement protrusions 312 whose importance is set high.

In an embodiment of the present disclosure, the controller 115 may be electrically connected to the plurality of moisture measurement protrusions 312, and the moisture amount may be determined based on the impedance measured between the first moisture measurement protrusion 315 and the second moisture measurement protrusion 316.

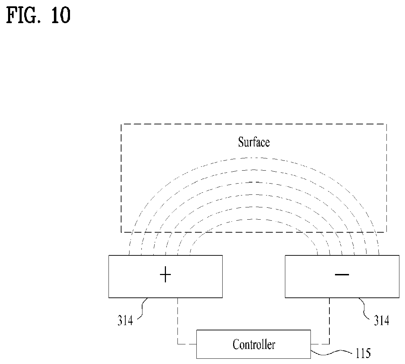

The impedance may be defined as the bioelectrical impedance measured at the user's scalp and the like as described above. FIG. 10 schematically shows a state in which the impedance is measured at the user's scalp and the like through the pair of moisture measurement protrusions 312 according to an embodiment of the present disclosure.

As described above, in an embodiment of the present disclosure, the pair of moisture measurement protrusions 312 may form the electric field on the scalp of the user by the mutual electric action.

The electric field may be amplified as the moisture amount of the scalp increases and may decrease as the moisture amount decreases. That is, when the electric field is formed by the pair of moisture measurement protrusions 312, the greater the moisture amount on the scalp, the lower the measured impedance, that is, the bioelectrical impedance, and the smaller the moisture amount, the higher the measured impedance.

The impedance measurement as described above may be performed by a current value, a voltage value, or the like generated between the pair of moisture measurement protrusions 312. For example, increase or decrease of the current value, the voltage value, or the like may be measured by increase or decrease of the impedance.

As described above, the change in the current value or the voltage value may correspond to the impedance formed in the electric field, that is, the bioelectrical impedance. The controller 115 may calculate or determine the moisture amount using the impedance measured through the change in the current value or the voltage value measured by the pair of moisture measurement protrusions 312.

The controller 115 may derive a moisture amount corresponding to a current impedance based on a pre-stored data map, or derive the moisture amount corresponding to the current impedance based on a pre-stored derivation equation.

The controller 115 may be disposed at one of the diffuser 200 and the main body 110 as described above, or may be disposed at each of the diffuser 200 and the main body 110. The controller 115 may utilize the moisture amount determined in the above manner variously to control the hair dryer 100.

For example, an embodiment of the present disclosure may further include the temperature adjuster 117 disposed on the main body 110 to adjust the temperature of the gas discharged through the gas outlet 150. The controller 115 may control the temperature adjuster 117 such that the temperature of the gas discharged through the gas outlet 150 increases as the moisture amount increases.

That is, when the moisture amount of the user's scalp or hair is high, it may be a situation in which the user requires scalp or hair drying. Accordingly, the controller 115 may control the temperature adjuster 117 to increase the gas temperature, thereby creating favorable conditions for drying the scalp or the hair.

On the other hand, when the moisture amount of the user's scalp or hair is small, it may be a situation in which the user does not require drying of the scalp or the hair, but rather is unpleasant with the discharge of the gas of the high temperature. Accordingly, the controller 115 may control the temperature adjuster 117 so as not to increase the gas temperature or so as to rather decrease the gas temperature.

In one example, an embodiment of the present disclosure may further include the fan 119 disposed to adjust the speed of the gas discharged through the gas outlet 150, and the controller 115 may control the fan 119 such that the speed of the gas discharged through the gas discharge unit 150 increases as the moisture amount increases.

When the moisture amount of the user's scalp or hair is high, it may be the situation in which the user requires the scalp or hair drying. Accordingly, the controller 115 may control the fan 119 to increase the speed of the gas discharged from the gas outlet 150, thereby creating the favorable conditions for drying the scalp or the hair.

On the other hand, when the moisture amount of the user's scalp or hair is small, it may be the situation in which the user does not require the drying of the scalp or the hair, but rather is unpleasant with the discharge of the gas of the high temperature. Accordingly, the controller 115 may control the fan 119 such that the speed of the gas discharged from the gas outlet 150 corresponds to a low speed.

In one example, an embodiment of the present disclosure may further include the light irradiator 260 disposed inside the diffusing case 210 to irradiate the light toward the discharge cover 300. The controller 115 may control the light irradiator 260 such that the amount of light irradiated by the light irradiator 260 increases as the moisture amount increases.

When the moisture amount of the user's scalp or hair is high, it may be the situation in which the user requires the scalp or hair drying. Accordingly, the controller 115 may control the light irradiator 260 to irradiate the light forwardly of the diffuser 200 or to provide the light of an amount increased than before, thereby creating the favorable conditions for drying the scalp or the hair.

On the other hand, when the moisture amount of the user's scalp or hair is small, it may be a situation in which the user does not require the drying of the scalp or the hair, but rather is unpleasant with temperature increase due to the light irradiation. Accordingly, the controller 115 may control the light irradiator 260 so as not to irradiate the light forwardly of the diffuser 200 or so as to provide the light of an amount reduced than before.

However, the control of the temperature adjuster 117, the fan 119, the light irradiator 260, and the like base on the moisture amount may not be necessarily limited as described above, and may be variously determined in a control strategic aspect for reflecting another request of the user or improving ease of use.

In one example, in an embodiment of the present disclosure, the light irradiator 260 may include the circuit board 265 and the light emitters 262 arranged on the circuit board 265 to irradiate the light, and the moisture measurement electrode 314 may be connected to the circuit board 265 to generate the voltage.

FIG. 9 shows the moisture measurement electrode 314 connected to the circuit board 265 of the light irradiator 260 according to an embodiment of the present disclosure. The moisture measurement electrode 314 may be disposed such that one end thereof may be electrically connected to the circuit board 265 and the other end thereof may penetrate the protrusion base 313 and is exposed to the outside from the front end of the protrusion base 313.

Accordingly, an embodiment of the present disclosure is advantageous in design because there is no need to construct a separate circuit for utilization of the moisture measurement protrusion 312 or the moisture measurement electrode 314, and is able to efficiently generate the voltage on the moisture measurement electrode 314.

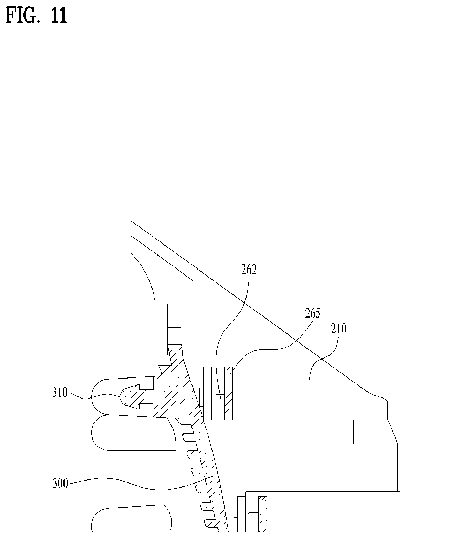

In one example, FIG. 11 shows a state in which the light emitter 262 of the light irradiator 260 is disposed rearward of the massage protrusion 310 of the discharge cover 300 according to an embodiment of the present disclosure. Referring to FIG. 11, in an embodiment of the present disclosure, the plurality of light emitters 262 may be arranged inside the diffusing case 210 to respectively face the plurality of massage protrusions 310.

The light irradiated from the light emitter 262 may be transmitted to the massage protrusion 310 through the gas discharge hole 305, or may be transmitted to the massage protrusion 310 by passing through the discharge cover 300 as the discharge cover 300 is made of a light transmissive material.

Accordingly, the light irradiated from the light emitter 262 may be transmitted to the scalp and the hair of the user through the massage protrusion 310, so that direct light transmission may be possible and the care effect of the scalp and the hair may be improved.

However, an embodiment of the present disclosure is not necessarily limited thereto. For example, some of the plurality of light emitters 262 may be respectively arranged rearward of the massage protrusions 310 and the others may be arranged rearward of the gas discharge hole 305 to irradiate the light.

Further, the plurality of light emitters 262 may be evenly distributed such that separation distances therebetween are uniform, or may be concentrated in some regions, regardless of the arrangement of the massage protrusions 310.

Although a specific embodiment of the present disclosure has been illustrated and described above, those of ordinary skill in the art to which the present disclosure pertains will appreciate that various modifications are possible within the limits without departing from the technical scope of the present disclosure provided by the following claims.

## Claims

1. A hair dryer comprising:
a main body (110) including a gas outlet (150) for discharging gas therethrough;
a handle (180) extending from the main body (110); and
a diffuser (200) removably coupled to the main body (110) to introduce the gas discharged from the gas outlet (150) therein and discharge the gas introduced therein to outside,
wherein the diffuser (200) includes:
a diffusing case (210) having a rear side (212) coupled to the main body (110), wherein the gas discharged from the gas outlet (150) is introduced into the diffusing case (210) through a gas inlet hole (215) defined at the rear side (212),
a discharge cover (300) disposed at a front side (211) of the diffusing case (210), wherein the discharge cover (300) includes a gas discharge hole (305) for discharging the gas introduced into the diffusing case (210) to outside,
wherein the discharge cover (300) includes a plurality of massage protrusions (310) protruding forward to press a target located in front of the discharge cover (300),
**characterized in that**
the plurality of massage protrusions (310) include a moisture measurement protrusion (312) disposed to measure a moisture amount of the target.

2. The hair dryer of claim 1, wherein the moisture measurement protrusion (312) includes:
a protrusion base (313) protruding forward to press the target; and
a moisture measurement electrode (314) disposed in the protrusion base (313), wherein at least a portion of the moisture measurement electrode (314) is exposed out of the protrusion base (313), and wherein the moisture measurement electrode (314) has an electrical polarity as a voltage is applied thereto.

3. The hair dryer of claim 2, wherein the moisture measurement electrode (314) extends to penetrate the protrusion base (313) along a longitudinal direction of the protrusion base (313) and is exposed to outside through an end of the protrusion base (313).

4. The hair dryer of claim 2, wherein the moisture measurement protrusion (312) includes a plurality of moisture measurement protrusions,
wherein the plurality of moisture measurement protrusions include:
a first moisture measurement protrusion (315) including a moisture measurement electrode having a first pole; and
a second moisture measurement protrusion (316) including a moisture measurement electrode having a second pole opposite to the first pole,
wherein the first moisture measurement protrusion (315) and the second moisture measurement protrusion (316) are arranged to be adjacent to each other.

5. The hair dryer of claim 4, wherein the plurality of massage protrusions includes (312) a plurality of pairs of moisture measurement protrusions, wherein each pair of moisture measurement protrusions includes the first moisture measurement protrusion (315) and the second moisture measurement protrusion (316).

6. The hair dryer of claim 4, further comprising:
a controller (115) electrically connected to the plurality of moisture measurement protrusions (312), wherein the controller (115) is configured to determine the moisture amount based on an impedance measured between the first moisture measurement protrusion (315) and the second moisture measurement protrusion (316).

7. The hair dryer of claim 6, further comprising:
a temperature adjuster (117) disposed on the main body (110) to adjust a temperature of the gas discharged through the gas outlet (150),
wherein the controller (115) is configured to control the temperature adjuster (117) such that the temperature of the gas discharged through the gas outlet (150) increases as the moisture amount increases.

8. The hair dryer of claim 6, further comprising:
a fan (119) disposed to adjust a speed of the gas discharged through the gas outlet (150),
wherein the controller (115) is configured to control the fan (119) such that the speed of the gas discharged through the gas outlet (150) increases as the moisture amount increases.

9. The hair dryer of claim 6, further comprising:
a light irradiator (260) disposed inside the diffusing case (210) to irradiate light toward the discharge cover (300),
wherein the controller (115) is configured to control the light irradiator (260) such that an amount of the light irradiated by the light irradiator (260) increases as the moisture amount increases.

10. The hair dryer of claim 9, wherein the light irradiator (260) includes a circuit board (265) and a light emitter (262) disposed on the circuit board (265) to emit light,
wherein the moisture measurement electrode (314) is connected to the circuit board (265) to receive the voltage.

11. The hair dryer of claim 10, wherein the light irradiator (260) includes a plurality of light emitters (262) arranged to respectively face the plurality of massage protrusions (310).

12. A diffuser (200) comprising:
a diffusing case (210) having a rear side removably coupled to a main body (110) of a hair dryer, wherein gas discharged from the main body (110) is introduced into the diffusing case (210) through a gas inlet hole (215) defined in the rear side (212); and
a discharge cover (300) disposed at a front side (211) of the diffusing case (210), wherein the discharge cover (300) includes a gas discharge hole (305) for discharging the gas introduced into the diffusing case (210)to outside,
wherein the discharge cover (300) includes a plurality of massage protrusions (310) protruding forward to press a target located in front of the discharge cover (300),
**characterized in that**
the plurality of massage protrusions (310) include a moisture measurement protrusion (312) disposed to measure a moisture amount of the target.

13. The diffuser of claim 12, wherein the moisture measurement protrusion (312) includes:
a protrusion base (313) protruding forward to press the target; and
a moisture measurement electrode (314) disposed in the protrusion base (313), wherein at least a portion of the moisture measurement electrode (314) is exposed out of the protrusion base (313), and wherein the moisture measurement electrode (314) has an electrical polarity as a voltage is applied thereto.

14. The diffuser of claim 13, wherein the moisture measurement protrusion (312) includes a plurality of moisture measurement protrusions,
wherein the plurality of moisture measurement protrusions include:
a first moisture measurement protrusion (315) including a moisture measurement electrode having a first pole; and
a second moisture measurement protrusion (316) including a moisture measurement electrode having a second pole opposite to the first pole,
wherein the first moisture measurement protrusion (315) and the second moisture measurement protrusion (316) are arranged to be adjacent to each other.

15. The diffuser of claim 14, wherein the plurality of massage protrusions (310) includes a plurality of pairs of moisture measurement protrusions, wherein each pair of moisture measurement protrusions includes the first moisture measurement protrusion (315) and the second moisture measurement protrusion (316).

## Patentansprüche

1. Haartrockner, der aufweist:
einen Hauptkörper (110), der einen Gasauslass (150) aufweist, um Gas hier hindurch auszugeben;
einen Griff (180), der sich vom Hauptkörper (110) aus erstreckt; und
einen Diffusor (200), der abnehmbar mit dem Hauptkörper (110) gekoppelt ist, um das aus dem Gasauslass (150) ausgegebene Gas darin einzuleiten und das darin eingeleitete Gas nach außen auszugeben,
wobei der Diffusor (200) aufweist:
ein Diffusionsgehäuse (210), das eine Rückseite (212) aufweist, die mit dem Hauptkörper (110) verbunden ist, wobei das von dem Gasauslass (150) ausgegebene Gas durch eine an der Rückseite (212) definierte Gaseinlassöffnung (215) in das Diffusionsgehäuse (210) eingeleitet wird,
eine Auslassabdeckung (300), die an einer Vorderseite (211) des Diffusionsgehäuses (210) angeordnet ist, wobei die Auslassabdeckung (300) eine Gasauslassöffnung (305) zum Auslassen des in das Diffusionsgehäuse (210) eingeleiteten Gases nach außen aufweist,
wobei die Auslassabdeckung (300) mehrere Massagevorsprünge (310) aufweist, die nach vorne hervorstehen, um ein vor der Auslassabdeckung (300) befindliches Ziel zu pressen,
**dadurch gekennzeichnet, dass**
die mehreren Massagevorsprünge (310) einen Feuchtigkeitsmessvorsprung (312) aufweisen, der zum Messen einer Feuchtigkeitsmenge des Ziels angeordnet ist.

2. Haartrockner nach Anspruch 1, wobei der Feuchtigkeitsmessvorsprung (312) aufweist:
eine Vorsprungsbasis (313), die nach vorne hervorsteht, um das Ziel zu pressen; und
eine Feuchtigkeitsmesselektrode (314), die in der Vorsprungsbasis (313) angeordnet ist, wobei mindestens ein Bereich der Feuchtigkeitsmesselektrode (314) von der Vorsprungsbasis (313) freiliegt und wobei die Feuchtigkeitsmesselektrode (314) eine elektrische Polarität aufweist, wenn eine Spannung daran angelegt wird.

3. Haartrockner nach Anspruch 2, wobei sich die Feuchtigkeitsmesselektrode (314) erstreckt, um durch die Vorsprungsbasis (313) entlang einer Längsrichtung des Vorsprungsbasis (313) hindurchzugehen und durch ein Ende der Vorsprungsbasis (313) nach außen freiliegt.

4. Haartrockner nach Anspruch 2, wobei der Feuchtigkeitsmessvorsprung (312) mehrere Feuchtigkeitsmessvorsprünge aufweist,
wobei die mehreren Feuchtigkeitsmessvorsprünge aufweisen:
einen ersten Feuchtigkeitsmessvorsprung (315), der eine Feuchtigkeitsmesselektrode mit einem ersten Pol aufweist; und
einen zweiten Feuchtigkeitsmessvorsprung (316), der eine Feuchtigkeitsmesselektrode mit einem zweiten Pol gegenüberliegend dem ersten Pol aufweist,
wobei der erste Feuchtigkeitsmessvorsprung (315) und der zweite Feuchtigkeitsmessvorsprung (316) angeordnet sind, um zueinander benachbart zu sein.

5. Haartrockner nach Anspruch 4, wobei die mehreren Massagevorsprünge mehrere Paare von Feuchtigkeitsmessvorsprüngen (312) aufweisen, wobei ein jedes Paar von Feuchtigkeitsmessvorsprüngen den ersten Feuchtigkeitsmessvorsprung (315) und den zweiten Feuchtigkeitsmessvorsprung (316) aufweist.

6. Haartrockner nach Anspruch 4, der ferner aufweist:
eine Steuereinheit (115), die elektrisch mit den mehreren Feuchtigkeitsmessvorsprüngen (312) verbunden ist, wobei die Steuereinheit (115) konfiguriert ist, die Feuchtigkeitsmenge basierend auf einer zwischen dem ersten Feuchtigkeitsmessvorsprung (315) und dem zweiten Feuchtigkeitsmessvorsprung (316) gemessenen Impedanz zu bestimmen.

7. Haartrockner nach Anspruch 6, der ferner aufweist:
einen Temperaturanpasseinrichtung (117), die am Hauptkörper (110) angeordnet ist, um eine Temperatur des durch den Gasauslass (150) ausgegebenen Gases anzupassen,
wobei die Steuereinheit (115) konfiguriert ist, die Temperaturanpasseinrichtung (117) zu steuern, dass die Temperatur des durch den Gasauslass (150) ausgegebenen Gases zunimmt, wenn die Feuchtigkeitsmenge zunimmt.

8. Haartrockner nach Anspruch 6, der ferner aufweist:
ein Gebläse (119), das angeordnet ist, eine Geschwindigkeit des durch den Gasauslass (150) ausgegebenen Gases anzupassen,
wobei die Steuereinheit (115) konfiguriert ist, das Gebläse (119) zu steuern, sodass die Geschwindigkeit des durch den Gasauslass (150) ausgegebenen Gases zunimmt, wenn die Feuchtigkeitsmenge zunimmt.

9. Haartrockner nach Anspruch 6, der ferner aufweist:
eine Lichtausstrahlungseinrichtung (260), die im Inneren des Diffusionsgehäuses (210) angeordnet ist, um Licht in Richtung der Auslassabdeckung (300) auszustrahlen,
wobei die Steuereinheit (115) konfiguriert ist, die Lichtausstrahlungseinrichtung (260) zu steuern, sodass eine Menge des von der Lichtausstrahlungseinrichtung (260) ausgestrahlten Lichts zunimmt, wenn die Feuchtigkeitsmenge zunimmt.

10. Haartrockner nach Anspruch 9, wobei die Lichtausstrahlungseinrichtung (260) eine Leiterplatte (265) und einen auf der Leiterplatte (265) angeordneten Lichtabstrahler (262) zum Abstrahlen von Licht aufweist,
wobei die Feuchtigkeitsmesselektrode (314) mit der Leiterplatte (265) verbunden ist, um die Spannung zu empfangen.

11. Haartrockner nach Anspruch 10, wobei die Lichtausstrahlungseinrichtung (260) mehrere Lichtabstrahler (262) aufweist, die angeordnet sind, um jeweils den mehreren Massagevorsprüngen (310) zugewandt zu sein.

12. Diffusor (200), der aufweist:
ein Diffusionsgehäuse (210), das eine Rückseite hat, die abnehmbar mit einem Hauptkörper (110) eines Haartrockners gekoppelt ist, wobei von dem Hauptkörper (110) ausgegebenes Gas durch eine in der Rückseite (212) definierte Gaseinlassöffnung (215) in das Diffusionsgehäuse (210) eingeleitet wird; und
eine Auslassabdeckung (300), die an einer Vorderseite (211) des Diffusionsgehäuses (210) angeordnet ist, wobei die Auslassabdeckung (300) eine Gasauslassöffnung (305) zum Auslassen des in das Diffusionsgehäuse (210) eingeleiteten Gases nach außen aufweist,
wobei die Auslassabdeckung (300) mehrere Massagevorsprünge (310) aufweist, die nach vorne hervorstehen, um ein vor der Auslassabdeckung (300) angeordnetes Ziel zu pressen,
**dadurch gekennzeichnet, dass**
die mehreren Massagevorsprünge (310) einen Feuchtigkeitsmessvorsprung (312) aufweisen, der zum Messen einer Feuchtigkeitsmenge des Ziels angeordnet ist.

13. Diffusor nach Anspruch 12, wobei der Feuchtigkeitsmessvorsprung (312) aufweist:
eine Vorsprungsbasis (313), die nach vorne hervorsteht, um das Ziel zu pressen; und
eine Feuchtigkeitsmesselektrode (314), die in der Vorsprungsbasis (313) angeordnet ist, wobei mindestens ein Bereich der Feuchtigkeitsmesselektrode (314) aus der Vorsprungsbasis (313) freigelegt ist, und wobei die Feuchtigkeitsmesselektrode (314) eine elektrische Polarität aufweist, wenn eine Spannung daran angelegt wird.

14. Diffusor nach Anspruch 13, wobei der Feuchtigkeitsmessvorsprung (312) mehrere Feuchtigkeitsmessvorsprünge aufweist,
wobei die mehreren Feuchtigkeitsmessvorsprünge aufweisen:
einen ersten Feuchtigkeitsmessvorsprung (315), der eine Feuchtigkeitsmesselektrode mit einem ersten Pol aufweist; und
einen zweiten Feuchtigkeitsmessvorsprung (316), der eine Feuchtigkeitsmesselektrode mit einem zweiten Pol gegenüberliegend dem ersten Pol aufweist,
wobei der erste Feuchtigkeitsmessvorsprung (315) und der zweite Feuchtigkeitsmessvorsprung (316) angeordnet sind, um zueinander benachbart zu sein.

15. Diffusor nach Anspruch 14, wobei die mehreren Massagevorsprünge (310) mehrere Paare von Feuchtigkeitsmessvorsprüngen aufweisen, wobei ein jedes Paar von Feuchtigkeitsmessvorsprüngen den ersten Feuchtigkeitsmessvorsprung (315) und den zweiten Feuchtigkeitsmessvorsprung (316) aufweist.

## Revendications

1. Sèche-cheveux comprenant :
un corps principal (110) incluant une sortie de gaz (150) pour décharger un gaz à travers celle-ci ;
une poignée (180) s'étendant depuis le corps principal (110) ; et
un diffuseur (200) couplé de manière amovible au corps principal (110) pour introduire le gaz déchargé depuis la sortie de gaz (150) à l'intérieur de celui-ci et pour décharger le gaz introduit à l'intérieur vers l'extérieur,
dans lequel le diffuseur (200) inclut :
un boîtier diffuseur (210) ayant un côté arrière (212) couplé au corps principal (110), le gaz déchargé depuis la sortie de gaz (150) étant introduit dans le boîtier diffuseur (210) à travers un trou d'entrée de gaz (215) défini au niveau du côté arrière (212),
une couverture de décharge (300) disposée au niveau d'un côté avant (211) du boîtier diffuseur (210), la couverture de décharge (300) incluant un trou de décharge de gaz (305) pour décharger le gaz introduit dans le boîtier diffuseur (210) vers l'extérieur,
dans lequel la couverture de décharge (300) inclut une pluralité de projections de massage (310) se projetant vers l'avant pour appuyer contre une cible située à l'avant de la couverture de décharge (300),
**caractérisé en ce que**
la pluralité de projections de massage (310) incluent une projection de mesurage d'humidité (312) disposée pour mesurer une quantité d'humidité de la cible.

2. Sèche-cheveux selon la revendication 1, dans lequel la projection de mesurage d'humidité (312) inclut :
une projection de base (313) se projetant vers l'avant pour appuyer contre la cible ; et
une électrode de mesurage d'humidité (314) disposée dans la base de projection (313), au moins une portion de l'électrode de mesurage d'humidité (314) étant exposée hors de la projection de base (313), et l'électrode de mesurage d'humidité (314) ayant une polarité électrique lorsqu'une tension est appliquée sur celle-ci.

3. Sèche-cheveux selon la revendication 2, dans lequel l'électrode de mesurage humidité (314) s'étend de façon à pénétrer dans la base de projection (313) le long d'une direction longitudinale de la base de projection (313) et est exposée vers l'extérieur à travers une extrémité de la base de projection (313).

4. Sèche-cheveux selon la revendication 2, dans lequel la projection de mesurage d'humidité (312) inclut une pluralité de projections de mesurage d'humidité,
dans lequel la pluralité de projections de mesurage d'humidité incluent :
une première projection de mesurage d'humidité (315) incluant une électrode de mesurage d'humidité ayant un premier pôle ; et
une seconde projection de mesurage d'humidité (316) incluant une électrode de mesurage d'humidité ayant un second pôle opposé au premier pôle,
dans lequel la première projection de mesurage humidité (315) et la seconde projection de mesurage d'humidité (316) sont agencées de façon à être adjacentes l'une à l'autre.

5. Sèche-cheveux selon la revendication 4, dans lequel la pluralité de projections de massage incluent une pluralité de paires de projections de mesurage d'humidité (312), chaque paire de projections de mesurage d'humidité incluant la première projection de mesurage d'humidité (315) et la seconde projection de mesurage d'humidité (316).

6. Sèche-cheveux selon la revendication 4, comprenant en outre :
un contrôleur (115) connecté électriquement à la pluralité de projections de mesurage d'humidité (312), le contrôleur (115) étant configuré pour déterminer la quantité d'humidité sur la base d'une impédance mesurée entre la première projection de mesurage d'humidité protrusion (315) et la seconde projection de mesurage d'humidité (316).

7. Sèche-cheveux selon la revendication 6, comprenant en outre :
un ajusteur de température (117) disposé sur le corps principal (110) de façon à ajuster une température du gaz déchargé à travers la sortie de gaz (150),
dans lequel le contrôleur (115) est configuré pour commander l'ajusteur de température (117) de sorte que la température du gaz déchargé à travers la sortie de gaz (150) augmente lorsque la quantité d'humidité augmente.

8. Sèche-cheveux selon la revendication 6, comprenant en outre :
un ventilateur (119) disposé de façon à ajuster une vitesse du gaz déchargé à travers la sortie de gaz (150), le contrôleur (115) étant configuré pour commander le ventilateur (119) de sorte que la vitesse du gaz déchargé à travers la sortie de gaz (150) augmente lorsque la quantité d'humidité augmente.

9. Sèche-cheveux selon la revendication 6, comprenant en outre :
un moyen d'irradiation de lumière (260) disposé à l'intérieur du boîtier diffuseur (210) pour irradier une lumière vers la couverture de décharge (300),
dans lequel le contrôleur (115) est configurée pour commander le dispositif d'irradiation de lumière (260) de sorte qu'une quantité de la lumière irradiée par le moyen d'irradiation de lumière (260) augmente lorsque la quantité d'humidité augmente.

10. Sèche-cheveux selon la revendication 9, dans lequel le moyen d'irradiation de lumière (260) inclut une carte à circuit (265) et un émetteur de lumière (262) disposé sur la carte à circuit (265) pour émettre de la lumière,
dans lequel l'électrode de mesurage d'humidité (314) est connectée à la carte à circuit (265) pour recevoir la tension.

11. Sèche-cheveux selon la revendication 10, dans lequel le moyen d'irradiation de lumière (260) inclut une pluralité d'émetteurs de lumière (262) agencés de façon à être tournés respectivement vers la pluralité de projections de massage (310).

12. Diffuseur (200) comprenant :
un boîtier diffuseur (210) ayant un côté arrière couplé de manière amovible au corps principal (110) d'un sèche-cheveux, un gaz déchargé depuis le corps principal (110) étant introduit dans le boîtier diffuseur (210) à travers un trou d'entrée de gaz (215) défini dans le côté arrière (212) ; et
une couverture de décharge (300) disposée au niveau d'un côté avant (211) du boîtier diffuseur (210),
dans lequel la couverture de décharge (300) inclut un trou de décharge de gaz (305) pour décharger le gaz introduit dans le boîtier diffuseur (210) vers l'extérieur,
dans lequel la couverture de décharge (300) inclut une pluralité de projections de massage (310) se projetant vers l'avant pour appuyer contre une cible située à l'avant de la couverture de décharge (300),
**caractérisé en ce que**
la pluralité de projections de massage (310) incluent une projection de mesurage d'humidité (312) disposée pour mesurer une quantité d'humidité de la cible.

13. Diffuseur selon la revendication 12, dans lequel la projection de mesurage d'humidité (312) inclut :
une projection base (313) se projetant vers l'avant pour appuyer contre la cible ; et
une électrode de mesurage d'humidité (314) disposée dans la base de projection (313), au moins une portion de l'électrode de mesurage d'humidité (314) étant exposée hors de la projection de base (313), et l'électrode de mesurage d'humidité (314) ayant une polarité électrique lorsqu'une tension est appliquée sur celle-ci.

14. Diffuseur selon la revendication 13, dans lequel la projection de mesurage d'humidité (312) inclut une pluralité de projections de mesurage d'humidité,
dans lequel la pluralité de projections de mesurage d'humidité incluent :
une première projection de mesurage d'humidité (315) incluant une électrode de mesurage d'humidité ayant un premier pôle ; et
une seconde projection de mesurage d'humidité (316) incluant une électrode de mesurage d'humidité ayant un second pôle opposé au premier pôle,
dans lequel la première projection de mesurage d'humidité (315) et la seconde projection de mesurage d'humidité (316) sont agencées pour être adjacentes l'une à l'autre.

15. Diffuseur selon la revendication 14, dans lequel la pluralité de projections de massage (310) incluent une pluralité de paires de projections de mesurage d'humidité, chaque paire de projections de mesurage d'humidité incluant la première projection de mesurage d'humidité (315) et la seconde projection de mesurage d'humidité (316).
